# EUROPEAN PATENT APPLICATION

(11) **EP 4 434 544 A1**
(43) Date of publication of application: **25.09.2024**
(21) Application number: 22894993.9
(22) Date of filing: 21.11.2022
(51) Int. Cl.: A61K 45/00, C12N 15/11, C07K 1/00, A61P 35/00

(54) **CD300LD INHIBITOR AND USE THEREOF IN PREPARATION OF TUMOR IMMUNOTHERAPY PRODUCT**

(30) Priority: 19.11.2021 CN 202111392144; 19.11.2021 CN 202111392205; 18.11.2022 WO PCT/CN2022/132932
(71) Applicant: Fudan University, Shanghai 200433 (CN); Center for Excellence in Molecular Cell Science, Chinese Academy of Sciences, Shanghai 200031 (CN)
(72) Inventor: LUO, Min, Shanghai 200031 (CN); ZHAO, Yun, Shanghai 200031 (CN); LU, Zhigang, Shanghai 200031 (CN)
(74) Representative: Canzler & Bergmeier Patentanwälte Partnerschaft mbB
(86) International application number: PCT/CN2022/133211
(87) International publication number: WO 2023/088464

(57) **Abstract**

The present invention relates to the field of biomedicine, and particularly relates to a CD300LD inhibitor and a use thereof in preparation of a tumor immunotherapy product. According to the present invention, the CD300LD inhibitor adjusts the migration and function of a PMN-MDSC cell by adjusting S100A8/A9 in a downstream signal pathway of the CD300LD inhibitor, and plays an important role in the establishment of a tumor microenvironment; an isolated polypeptide of the present invention is used as a mechanism of using CD300LD-ECD protein competitiveness to inhibit CD300LD, so that the purpose of inhibiting tumor development is achieved; moreover, CD300LD-ECD and a PD1 antibody have an antitumor synergistic effect.

## Description

### FIELD OF DISCLOSURE

The present disclosure relates to the technical field of biomedicine, and in particular to a CD300LD inhibitor and a use thereof in the preparation of tumor immunotherapy products.

### DESCRIPTION OF RELATED ARTS

Immunotherapy has significantly enhanced the efficacy of clinical tumor treatments. However, immune checkpoint blockade therapies, including those targeting PD1, CTLA4, etc., demonstrate favorable therapeutic effects only in certain patients. The majority of patients do not benefit from these therapies, and the long-term benefit rate remains relatively low. Exploring a new immune checkpoint molecule is a key issue for current tumor immunotherapy. Polymorphonuclear myeloid-derived suppressor cells (PMN-MDSCs) are an important cell population in the tumor immune myeloid lineage. PMN-MDSC is a class of pathologically induced neutrophils widely present in various tumors. They have the ability to suppress the functions of effector cells like T cells and NK cells, thus promoting tumor development, infiltration, and metastasis. They also play a crucial role in creating a tumor immunosuppressive microenvironment.

### SUMMARY OF THE PRESENT INVENTION

The present disclosure provides a CD300LD inhibitor and a use thereof in the preparation of tumor immunotherapy products.

The present disclosure provides a use of CD300LD gene as a target in the preparation of tumor preventive, diagnostic, or therapeutic drugs.

The present disclosure also provides a use of the CD300LD gene and PD1 together as targets in the preparation of tumor preventive, diagnostic, or therapeutic drugs.

The present disclosure also provides a use of a CD300LD inhibitor in the preparation of products having at least one of the following effects:
tumor prevention, diagnosis, or treatment;
regulation of myeloid cell activity and/or migration;
regulation of T cell activity; and
regulation of CD300LD downstream signaling pathway.

The present disclosure also provides an isolated polypeptide, wherein the isolated polypeptide comprises a functional fragment of a CD300LD protein, and the functional fragment of the CD300LD protein is an extracellular region of the CD300LD protein.

In one embodiment, the extracellular region of the CD300LD protein is derived from a mammalian CD300LD protein or a mutant thereof. The mammal is preferably selected from a rodent, an even-toed ungulate, an odd-toed ungulate, a a lagomorph, and a primate. The rodent is preferably a rat. The primate is preferably a monkey, an ape, or a human.

In one embodiment, the amino acid sequence of the extracellular region of CD300LD is shown as SEQ ID NO. 17 or SEQ ID NO. 31.

Another aspect of the present disclosure provides a nucleic acid construct encoding the isolated polypeptide.

In one embodiment, the nucleic acid construct comprises a polynucleotide shown as SEQ ID NO. 16 or SEQ ID NO.30.

In certain embodiments of the present disclosure, a nucleotide sequence of the nucleic acid construct is shown as SEQ ID NO.19 or SEQ ID NO.32.

Another aspect of the present disclosure provides a cell line, comprising the nucleic acid construct, or a genome of the cell line has an exogenous polynucleotide shown as SEQ ID NO. 16 or SEQ ID NO. 30.

The present disclosure further provides a method for the preparation of the isolated polypeptide, comprising the following steps:
a) culturing a cell line as described above under expression conditions, so as to express the isolated polypeptide;
b) isolating and purifying the polypeptide described in step a).

The present disclosure further provides a use of the isolated polypeptide in the preparation of tumor preventive, diagnostic, or therapeutic drugs.

In certain embodiments of the present disclosure, the use includes the preparation of a product for the prevention, diagnosis, or treatment of tumors by using the isolated polypeptide and an anti-PD1 antibody.

The tumor is a CD300LD overexpressing tumor; preferably, the tumor is selected from an intestinal cancer, a lung cancer, a liver cancer, a breast cancer, an esophageal cancer, a head and neck cancer, a skin cancer, a kidney cancer, a leukemia, a cervical cancer, a colon cancer, a hepatocellular carcinoma, an ovarian serous cystadenocarcinoma, an endometrial carcinoma, a thyroid cancer, a cutaneous melanoma, a lung adenocarcinoma, a head and neck squamous cell carcinoma, a glioblastoma multiforme, a prostate cancer, a thymic carcinoma, a brain low-grade glioma, a rectal adenocarcinoma, a pheochromocytoma and paraganglioma, an esophageal carcinoma, a renal clear cell carcinoma, a bladder urothelial carcinoma, a renal papillary cell carcinoma, a pancreatic cancer, a stomach cancer, a chromophobe renal cell carcinoma, a breast invasive carcinoma, a lung squamous cancer, a sarcoma, and an acute myeloid leukemia.

The present disclosure also provides a composition, comprising the CD300LD inhibitor. The composition has any one or more of the following effects:
tumor prevention, diagnosis, or treatment;
regulation of myeloid cell activity and/or migration;
regulation of T cell activity; and
regulation of CD300LD downstream signaling pathway.

The present disclosure also provides a pharmaceutical composition, comprising the CD300LD inhibitor. The CD300LD inhibitor is the isolated polypeptide.

The present disclosure also provides a method for treating a CD300LD overexpressing disease, comprising administering to an individual a therapeutically effective amount of the isolated polypeptide or the composition.

As described above, the present disclosure provides CD300LD-ECD and its use in the preparation of tumor immunotherapy products. The present disclosure has the following beneficial effects: a new immune checkpoint molecule specific to PMN-MDSC, CD300LD, is provided, which specifically and efficiently targets and regulates PMN-MDSC, proposing a new target and idea for tumor immunotherapy through PMN-MDSC. The provided CD300LD-ECD can competitively inhibit CD300LD, thus significantly inhibiting the development of many kinds of tumors, and shows obvious anti-tumor synergistic effect with PD1 antibody, providing a new targeting drug for tumor immunotherapy.

### BRIEF DESCRIPTION OF THE DRAWINGS

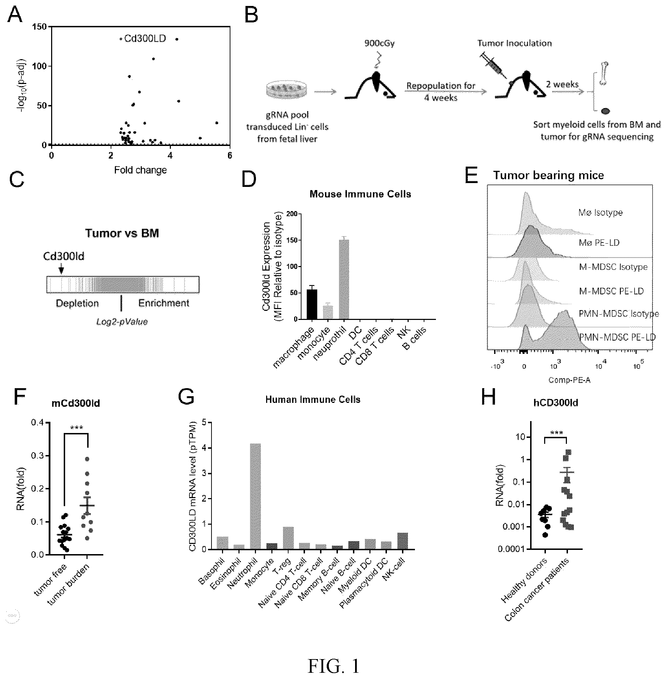

FIGs. 1A-1H show an identification of CD300LD gene and its gene expression profile in immune cells according to Embodiment 1 of the present disclosure. FIG. 1A represents differentially expressed genes in PMN-MDSC versus neutrophils; FIG. 1B is a schematic diagram of a screening process of in vivo screening for myeloid cell surface receptors using Crispr-Cas9 and B16-F10 tumor models; FIG. 1C represents a screening of enriched genes (C), where CD300LD gRNA is specifically absent in tumors compared to bone marrow of tumor-bearing mice; FIG. ID represents a flow analysis showing that in normal mouse immune cells, CD300LD is specifically expressed in myeloid cells in the leukocytes, in particular, is specifically highly expressed in neutrophils (CD45⁺CD11b⁺Ly6G⁺) (n=3); FIG. 1E represents a flow analysis showing CD300LD expression in intratumoral myeloid cells, including macrophages Mø (CD45⁺CD11b⁺F4/80⁺), mononuclear myeloid-derived suppressor cells M-MDSC (CD45⁺CD11b⁺Ly6c⁺ly6G), and polymorphonuclear myeloid-derived suppressor cell PMN-MDSC (CD45⁺CD11b⁺Ly6c^{low}ly6G⁺), where CD300LD is specifically highly expressed in PMN-MDSC; FIG. 1F represents a qRT-PCR showing mRNA levels of CD300LD in PMN-MDSC of tumor-bearing mice and neutrophils of normal mice; FIG. 1G represents an analysis of Human Protein Atlas database showing that CD300LD gene is predominantly expressed in blood/immune tissues, and is mainly expressed on neutrophils at the cellular subtype level; FIG. 1H represents a qRT-PCR showing quantification of CD300LD gene in peripheral blood granulocytes of healthy individuals and colon cancer patients; where two-tailed t-test, * p<0.05, ** p<0.01, *** p<0.001 are used.

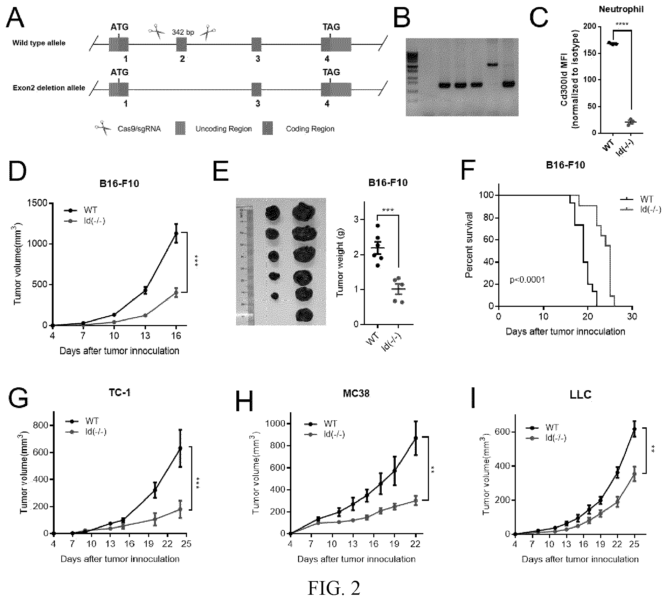

FIGs. 2A-2I show that CD300LD knockout inhibits the development of multiple tumors according to Embodiment 2 of the present disclosure. FIG. 2A is a schematic diagram of the construction of CD300LD KO mice; FIG. 2B represents genotyping results of CD300LD KO mice; FIG. 2C represents a flow analysis showing CD300LD expression in neutrophils of CD300LD WT mice and CD300LD KO mice; FIG. 2D represents tumor growth curves under a melanoma (B16-F10) tumor model of CD300LD WT mice and CD300LD KO mice (n=11); FIG. 2E represents 20-day tumor sizes under a melanoma (B16-F10) tumor model of CD300LD WT mice and CD300LD KO mice (n=11); FIG. 2F represents mouse survival curves under a melanoma (B16-F10) tumor model of CD300LD WT mice and CD300LD KO mice (n=11); FIG. 2G represents tumor growth curves under a TC-1 cervical cancer model of CD300LD WT mice and CD300LD KO mice (n=6); FIG. 2H represents tumor growth curves under a MC-38 colon cancer model of CD300LD WT mice and CD300LD KO mice (n=6); FIG. 2I represents tumor growth curves under a LLC lung cancer model of CD300LD WT mice and CD300LD KO mice (n=6); where two-tailed t-test, * p<0.05, ** p<0.01, *** p<0.001 are used.

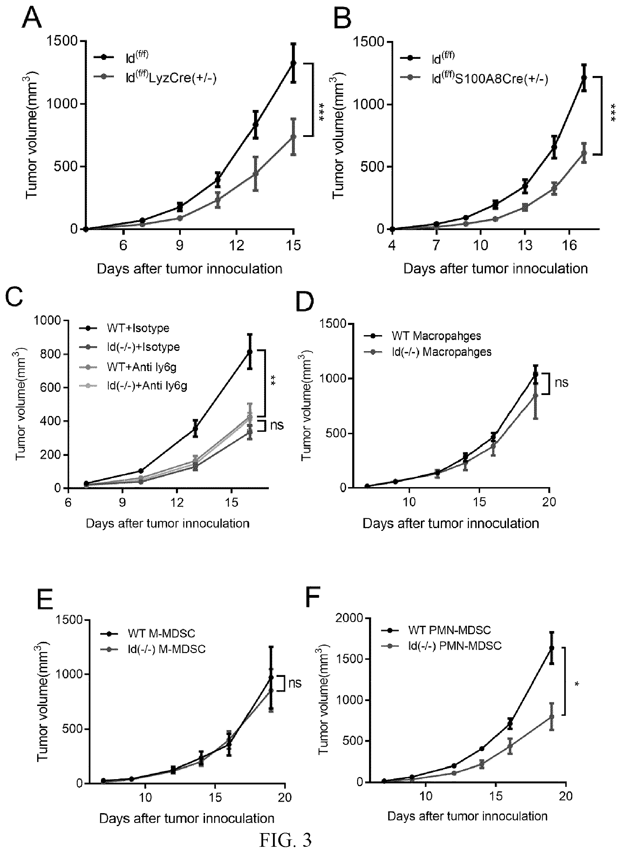

FIGs. 3A-3F show that the CD300LD knockout inhibits the tumor development via PMN-MDSC according to Embodiment 3 of the present disclosure. FIG. 3A represents that the CD300LD knockout in myeloid cells under a LyzCre condition significantly inhibits the development of B16-F10 tumor (n=6); FIG. 3B represents that the CD300LD knockout in PMN-MDSC in myeloid cells under an S100A8Cre condition significantly inhibits the development of B16-F10 tumor (n=9); FIG. 3C represents a clearance of PMN-MDSC in mice through Ly6g antibodies, which significantly inhibits the development of B16-F10 tumor in wild-type mice, and has no significant effect on the inhibition of B16-F10 tumor development in CD300LD knockout mice; FIG. 3D-3F represent growth curves of mice, where before being subcutaneously inoculated to wild-type mice, isolated macrophages (FIG. 3D), mononuclear myeloid-derived suppressor cells (FIG. 3E), and PMN-MDSC (FIG. 3F) from wild-type mice or CD300LD KO mice under the B16-F10 tumor model are mixed with B16-F10 cells at a ratio of 1:1, with only the presence of CD300LD in polymorphonuclear myeloid-derived suppressor cells resulting in differences in tumor growth;where two-tailed t-test, * p<0.05, ** p<0.01, *** p<0.001 are used.

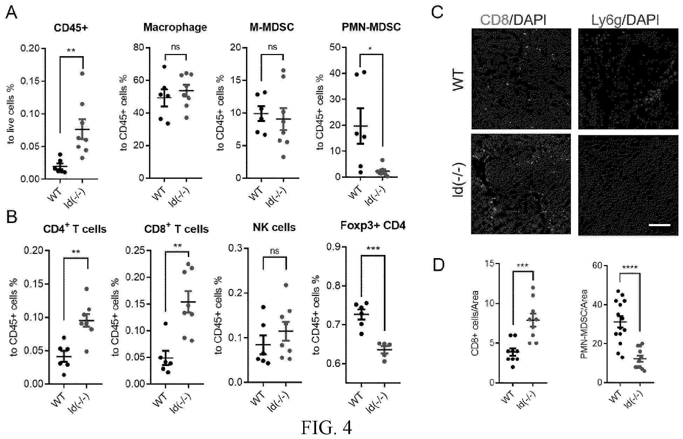

FIGs. 4A-4D show flow analysis results indicating that the CD300LD knockout improves the tumor immune microenvironment according to Embodiment 4 of the present disclosure. FIG. 4A represents flow analysis results showing proportions of leukocytes (CD45⁺) and major myeloid cell subsets in B16-F10 tumor; FIG. 4B represents flow analysis results showing the changes in the proportions of each lymphocyte subpopulation in B16-F10 tumor; FIG. 4C and FIG. 4D represent immunofluorescence analysis results of intratumoral PMN-MDSC (Ly6g⁺) and CD8⁺ T cells in B16-F10 tumor, where FIG. 4C is the immunofluorescence result (Bar=200um) and FIG. 4D is the data statistics of immunofluorescence analysis; where two-tailed t-test, * p<0.05, ** p<0.01, *** p<0.001 are used.

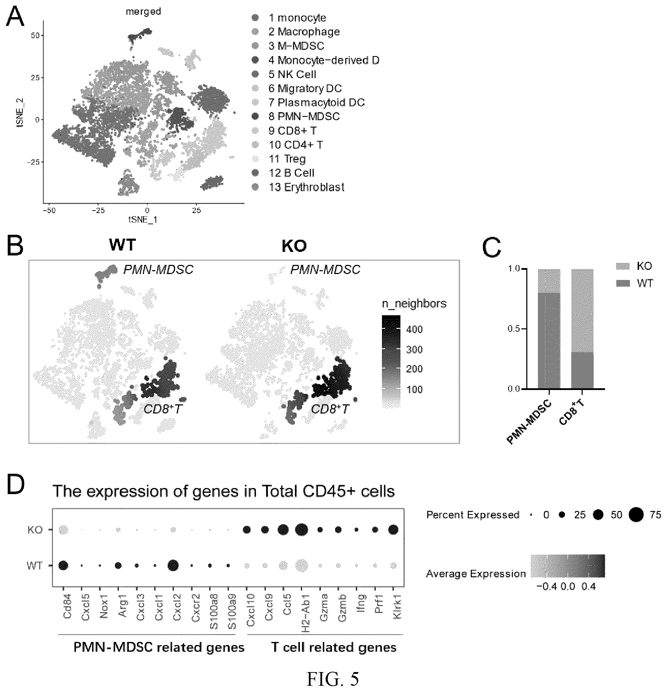

FIGs. 5A-5D show single-cell sequencing results indicating the CD300LD knockout improves tumor immune microenvironment according to Embodiment 4 of the present disclosure. FIG. 5A represents subtyping of immune cells in the B16-F10 tumor immune microenvironment by using single-cell sequencing, where FIG. 5A is a tSNE plot; FIG. 5B and FIG. 5C represent immune cell distribution in the B16-F10 tumor microenvironment of CD300LD WT mice and CD300LD KO mice, where PMN-MDSC in the KO group are significantly reduced and CD8⁺ T cells in the KO group are significantly increased, FIG. 5B is a tSNE plot, and FIG. 5C is a diagram showing proportions of the corresponding cells in WT and KO mice; FIG. 5D represents expression levels of PMN-MDSC function-related genes, as well as T-cell recruitment and function-related genes in immune cells of the WT and KO groups, where expression level of PMN-MDSC function-related genes is significantly down-regulated in the KO group, and expression level of T-cell infiltration and function-related genes is significantly increased; where two-tailed t-test, * p<0.05, ** p<0.01, *** p<0.001 are used.

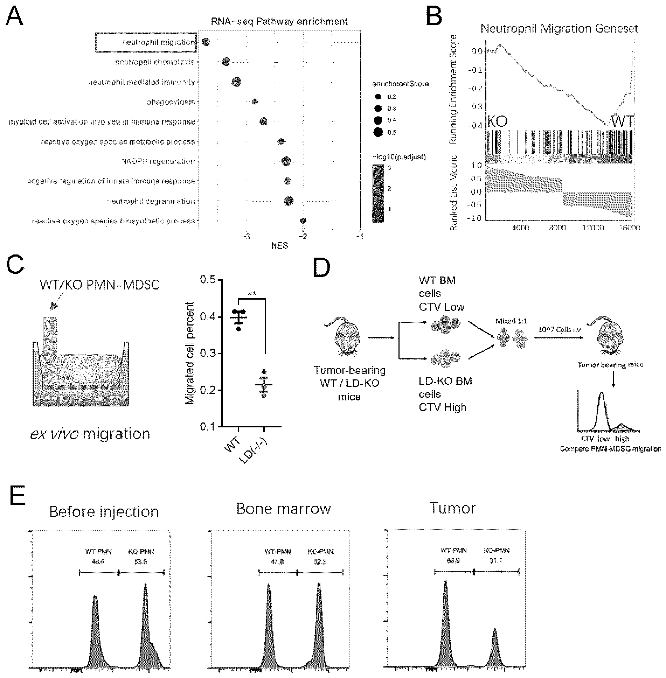

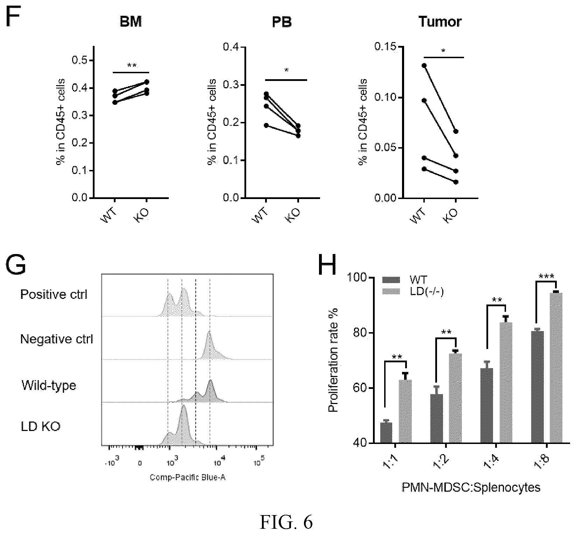

FIGs. 6A-6H shows that CD300LD-KO significantly supresses migration and function of PMN-MDSC according to Embodiment 5 of the present disclosure. FIG. 6A represents RNAseq analysis showing a gene expression profile of PMN-MDSC in CD300LD WT and CD300LD KO, and GO analysis revealing a significant difference in granulocyte migration signaling pathways; FIG. 6B represents GSEA analysis, revealing that granulocyte migration-related genes are significantly enriched in PMN-MDSC of CD300LD WT; FIG. 6C represents in vitro migration experiments of isolated PMN-MDSC from tumor-bearing CD300LD WT mice and CD300LD KO mice, where the left is a schematic diagram and the right is experimental statistics (n=3); FIG. 6D-FIG. 6F represent in vivo migration experiments of isolated PMN-MDSC from tumor-bearing CD300LD WT mice and CD300LD KO mice, where FIG. 6D is a schematic diagram of the procedure; FIG. 6E represents flow analysis plots and FIG. 6F represents statistical results of WT PMN-MDSC and KO PMN-MDSC in bone marrow and tumor before and after injection; FIG. 6G and FIG. 6H represent the inhibition ability of CD300LD WT PMN-MDSC and CD300LD KO PMN-MDSC towards T cells, where FIG. 6G is a flow diagram and FIG. 6H is the statistical analysis (n=3); where two-tailed t-tests, * p<0.05, ** p<0.01, *** p<0.001 are used.

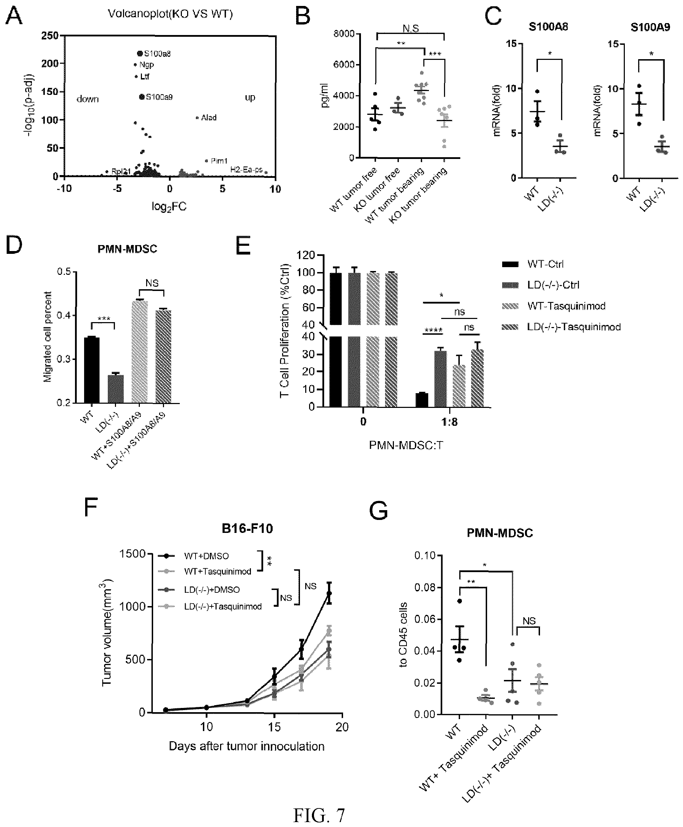

FIGs. 7A-7G show that CD300LD regulates migration and function of PMN-MDSC through S100A8/A9 according to Embodiment 6 of the present disclosure. FIG. 7A represents a RNAseq analysis showing differential genes between CD300LD WT PMN-MDSC and CD300LD KO PMN-MDSC, where the expression of PMN-MDSC-related genes S100A8/A9 is significantly downregulated in the CD300LD KO group; FIG. 7B represents an ELISA analysis showing S100A8/A9 levels in serum of WT and KO tumor-bearing mice; FIG. 7C represents a qRT-PCR analysis showing S100A8/A9 expression in CD300LD WT PMN-MDSC and CD300LD KO PMN-MDSC (n=3); FIG. 7D represents an effect of S100A8/A9 protein on the in vitro migration ability of PMN-MDSC (n=3); FIG. 7E represents an effect of S100A8/A9 inhibitor Tasquinimod on T cell proliferation inhibition in WT PMN-MDSC and KO PMN-MDSC(n=3); FIG. 7F and FIG. 7G represent tumor growth curves (FIG. 7F) and proportion of intratumoral PMN-MDSC (FIG. 7G) after injecting Tasquinimod, an inhibitor of S100A8/A9, intraperitoneally to tumor-bearing mice (n=45); where two-tailed t-test, * p<0.05, ** p<0.01, *** p<0.001 are used.

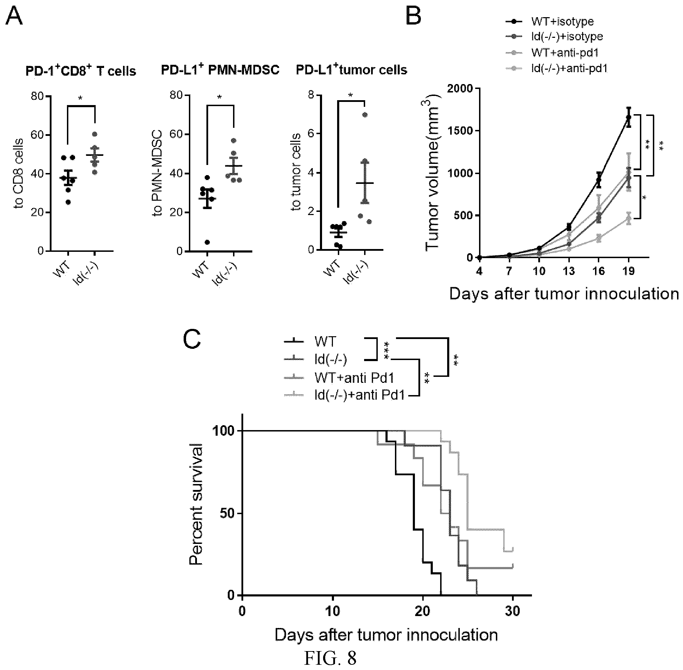

FIGs. 8A-8C show that the CD300LD knockout has an anti-tumor synergistic effect with PD antibodies according to Embodiment 7 of the present disclosure. FIG. 8A represents proportions of PD1⁺CD8⁺ cells, PD-L1⁺ PMN-MDSC cells, and PDL1⁺ tumor cells in CD300LD WT mouse tumor and CD300LD KO mouse tumor; FIG. 8B represents tumor growth curves after CD300LD WT and KO mice being inoculated with B16-F10 tumor cells, where a PD1 antibody treatment is performed on day 7 with 200 µg/mouse, 2 days once for a total of 3 times; FIG. 8C represents mouse survival curves (n=11 -15); where two-tailed t-test, * p<0.05, ** p<0.01, *** p<0.001 are used.

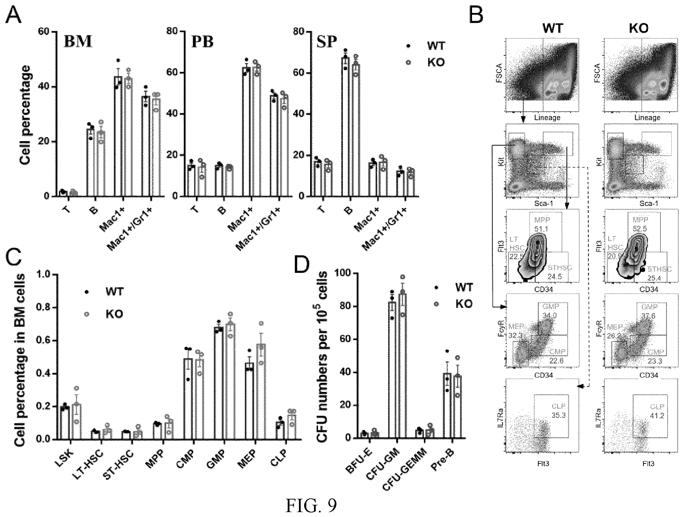

FIGs. 9A-9D show that the CD300LD knockout has no significant effect on immune and hematopoietic development according to Embodiment 8 of the present disclosure. FIG. 9A represents flow analysis statistics of immune cell ratios of bone marrow (BM), peripheral blood (PB), and T (CD3⁺), B (B220⁺), myeloid (Mac1⁺), and neutrophil (Mac1⁺Gr1⁺) in CD300LD WT mice and CD300LD KO mice (n=3); FIG. 9B and FIG. 9C represent flow analysis plots (B) and flow analysis statistics (C) of HSC and all precursor cells in the bone marrow of CD300LD WT mice and CD300LD KO mice (n=3); FIG. 9D represents clone formation ability of HSC in CD300LD WT mice and CD300LD KO mice (n=3); where two-tailed t-tests, * p<0.05, ** p<0.01, *** p<0.001 are used.

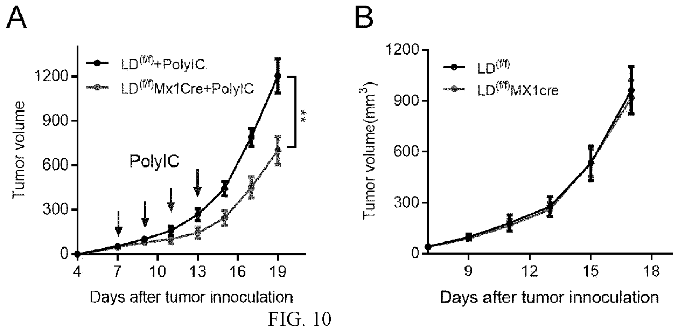

FIGs. 10A-10B show that induced knockout of CD300LD is able to inhibit the development of established tumors and can serve as a potent tumor therapeutic target according to Embodiment 9 of the present disclosure. FIG. 10A represents tumor growth curves, where the B16-F10 tumor model is established in CD300LD^{(f/f)}/Mx1-Cre and CD300LD^{(f/f)} mice, PolyI:C is injected on day 7 during tumor development to induce CD300LD knockout, and tumor development is significantly inhibited in CD300LD^{(f/f)}/Mx1-Cre mice (n=6); FIG. 10B represents a control of FIG. 10A, where mice were the same as those in FIG. 10A, and no significant difference in tumor growth is shown between mice in FIG. 10A and FIG. 10B without PolyI:C injection (n=6); where two-tailed t-tests, * p<0.05, ** p<0.01, *** p<0.001 are used.

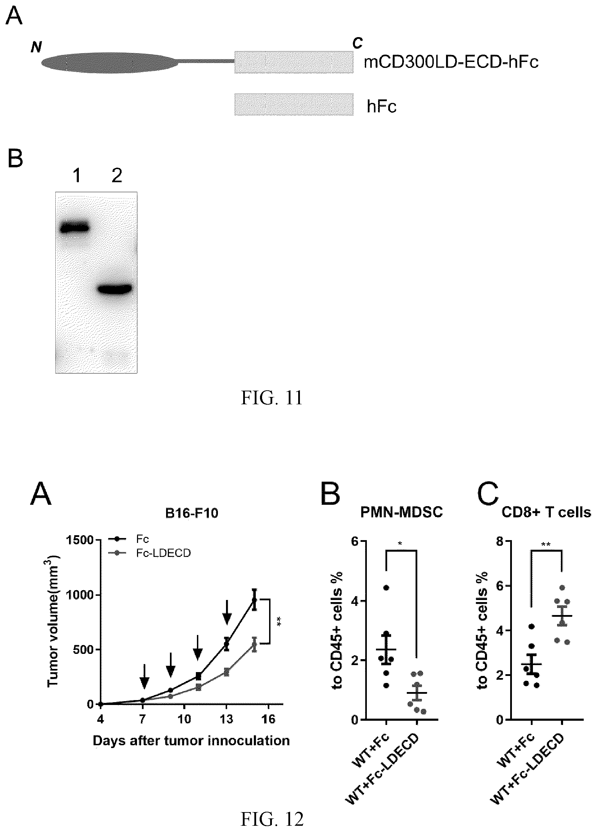

FIGs. 11A-11B show a purification of mouse CD300LD-ECD protein. FIG. 11A is a schematic diagram showing mCD300LD-ECD-hFc protein and hFc protein which serves as a control; FIG. 11B represents SDS-PAGE of purified proteins, where 1 is mCD300LD-ECD-hFc and 2 is hFc protein.
FIGs. 12A-12C show that mouse CD300LD-ECD significantly inhibits the development of the B16-F10 tumor model. FIG. 12A represents tumor growth curves of mice in different groups, where WT mice are inoculated with B16-F10 tumor cells subcutaneously, and are injected with proteins via the tail vein on day 7 after inoculation with every two days for a total of four times; FIG. 12B and FIG. 12C represent ratios of PMN-MDSC and CD8+ T cells in the tumors, where the tumor tissues are obtained from mice in different groups in FIG. 12A on day 15; where *p<0.05, ** p<0.01, *** p<0.001 are used.

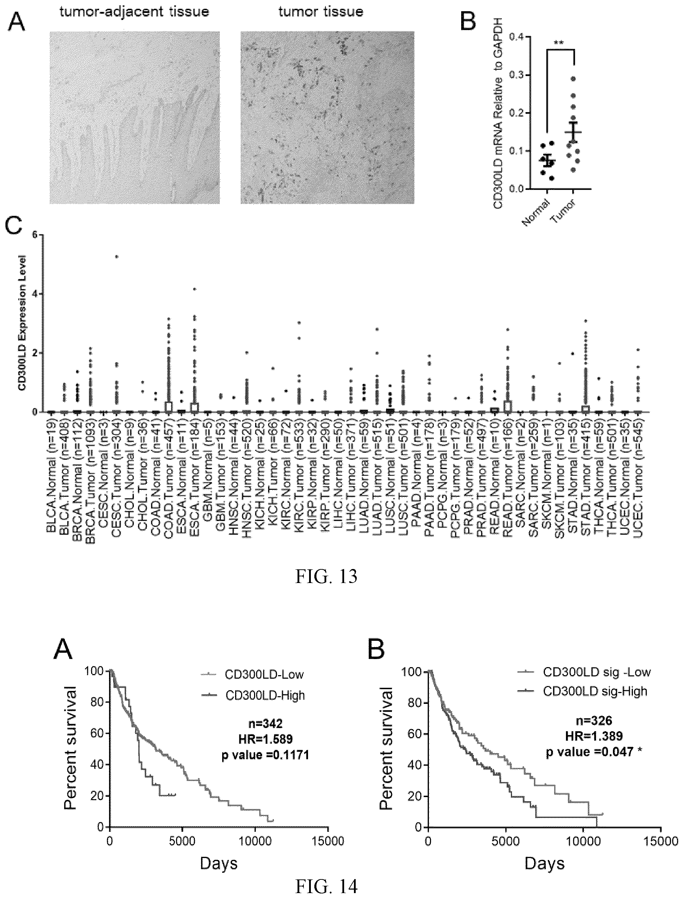

FIGs. 13A-13C show that an upregulation of hCD300LD expression in tumor patients. FIG. 13A represents immunohistochemical staining of CD300LD in melanoma tumor samples (Bar=200um); FIG. 13B represents expression level of CD300LD mRNA in peripheral blood immune cells of patients having intestinal cancer; FIG. 13C represents analysis of CD300LD mRNA expression levels in tumor samples (red) and normal tissues (blue) from different tumor types in the TCGA database, where normal tissues serve as a control; where *p<0.05, ** p<0.01, *** p<0.001 are used.
FIGs. 14A-14B show that an expression level of hCD300LD is negatively correlated with the survival of patients with melanoma. FIG. 14A represents a correlation analysis between CD300LD and patient survival rate; FIG. 14B represents a correlation analysis between CD300LD signature gene and patient survival rate.

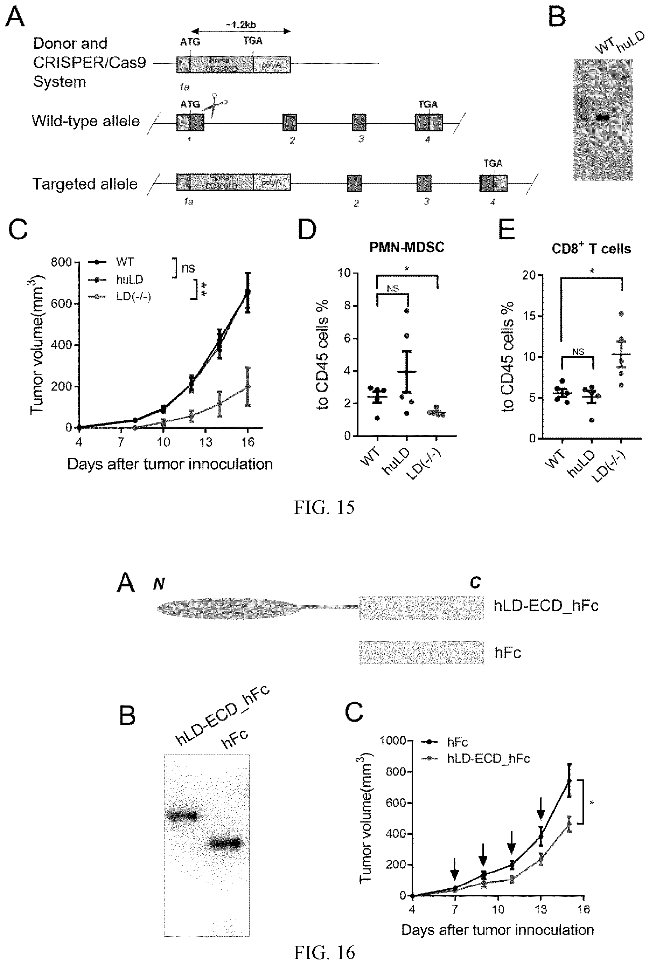

FIGs. 15A-15E show a construction of hCD300LD humanized mice. FIG. 15A represents a schematic diagram of hCD300LD humanized mouse construction. FIG. 15B represents a PCR analysis of genotypes of wild-type and humanized mice. FIG. 15C represents tumor growth curves of wild-type mice, CD300LD knockout mice, and CD300LD humanized mice, where these mice are inoculated with B16-F10 cells subcutaneously; FIG. 15D represents a ratio of PMN-MDSC in tumors, and FIG. 15E represents a ratio of CD8⁺ T cells in tumors, where the tumors are isolated on day 16; where *p<0.05, ** p<0.01, *** p<0.001 are used.
FIGs. 16A-16C show that hCD300LD-ECD significantly inhibits tumor development. FIG. 16A represents a schematic diagram of hCD300LD-ECD-hFc protein and hFc protein serving as a control; FIG. 16B represents SDS-PAGE of purified proteins, where 1 is hCD300LD-ECD-hFc and 2 is hFc protein; FIG. 16C represents tumor growth curves of mice in different groups, where CD300LD humanized mice are subcutaneously inoculated with B16-F10 tumor cells, and are injected with proteins via the tail vein on day 7 after inoculation with every two days for four times; where *p<0.05, ** p<0.01, *** p<0.001 are used.

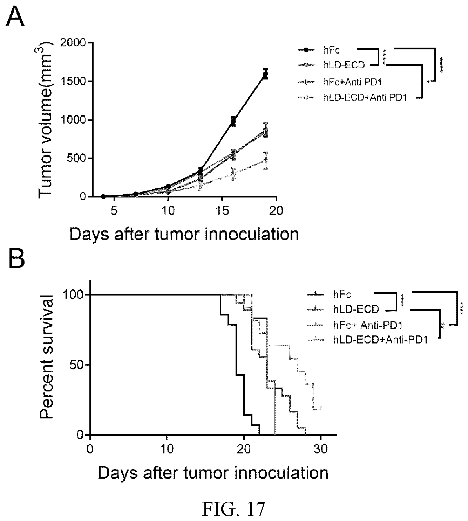

FIGs. 17A-17B show survival curves of mice indicating that hCD300LD-ECD significantly prolongs lifetime of mice, where B16-F10 cells are inoculated subcutaneously into CD300LD humanized mice, drug treatment is performed during the corresponding time, hCD300LD-ECD-hFc or hFc is injected via the tail vein on day 7 after inoculation with a dose of 200 µg protein/mouse, every 2 days for a total of 4 times; and PD1 antibody is injected via the tail vein on day 8 after inoculation with a dose of 200 µg protein/mouse, every 2 days for a total of 3 times. FIG. 17A represents tumor growth curves; FIG. 17B represents survival curves of mice; where *p<0.05, ** p<0.01, *** p<0.001 are used.

### DETAILED DESCRIPTION OF THE EMBODIMENTS

Unless otherwise defined hereinafter, all technical and scientific terms referred to in the present disclosure have the meaning commonly understood by those skilled in the art to which the present disclosure belongs.

The term "peptide" used herein refers to a polymer of amino acids without a specific length. Accordingly, peptides, oligopeptides, and proteins are included in the definition of "polypeptide" and are used interchangeably throughout the specification as well as in the claims. The term "polypeptide" does not exclude post-translational modifications, which include, but are not limited to, phosphorylation, acetylation, glycosylation, and the like.

The term "isolated polypeptide" means that the polypeptide is not in a living organism, e.g., human or mouse. The isolated polypeptide may be a part of the composition or kit. The isolated polypeptide is preferably purified.

The inventors of this application have been devoted to finding key checkpoint molecules that regulate PMN-MDSC. Since PMN-MDSC and neutrophils are similar in molecular typing but opposite in function, transcriptome sequencing is preformed on PMN-MDSC and neutrophils to analyze the differences in membrane protein expressions, meanwhile, a sgRNA library is constructed for PMN-MDSC membrane proteins, and an in vivo screening using CRISPR-Cas9 is performed in combination with a mouse tumor model. Both strategies point to a membrane protein, namely CD300LD. Human and murine CD300LD are specifically expressed on neutrophils/PMN-MDSC, and expression is significantly up-regulated in the latter after tumor engraftment. CD300LD knockout significantly inhibits tumor progression under a variety of models including melanoma, colon, breast, and lung cancers. Using conditional knockout of CD300LD or Ly6G antibody to remove PMN-MDSC in vivo, as well as isolation of different myeloid cell subpopulations for xenograft tumor model experiments, it has been demonstrated that PMN-MDSC specifically regulates tumor immunity through CD300LD. Therefore, CD300LD is a novel PMN-MDSC-specific tumor immunosuppressive receptor and a potent immune checkpoint molecule.

Mechanistic studies reveal that CD300LD knockout leads to a significant improvement in the tumor immunosuppressive microenvironment, including a dramatic reduction in PMN-MDSC, a significant increase in CD8+ T cell infiltration, a significant downregulation of PMN-MDSC migration and function-related pathways, and a significant upregulation of T-cell migration and killing-related signaling pathways. Further studies reveal that CD300LD regulates tumor migration and T cell suppressive function of PMN-MDSC through downstream S100A8/A9. In conclusion, CD300LD knockout regulates the recruitment and function of PMN-MDSC through S100A8/A9, which significantly improves the tumor immunosuppressive microenvironment and produces broad-spectrum antitumor effects.

In terms of safety, the knockout of CD300LD does not affect the normal development of the body and the immune system, which is unlike CSF1R and CD47. Regarding CSF1R and CD47, CSF1R is widely expressed in myeloid cells, and the knockout of CSF1R is lethal to the organism, the knockout of CD47 has strong side effects. In terms of efficacy, CD300LD knockout inhibits the development of various tumor models and shows a significant anti-tumor synergistic effect with PD1 antibody; more importantly, CD300LD knockout is also able to significantly inhibit tumor development after the establishment of a tumor. In summary, CD300LD, as a novel target, is safe and has excellent anti-tumor efficacy.

Based on the above studies, the present disclosure provides a use of CD300LD gene as a target in the preparation of drugs for tumor prevention, diagnosis, or treatment for the first time.

The use of CD300LD gene as a target in the preparation of drugs for tumor prevention, diagnosis, or treatment, specifically refers to: taking the CD300LD gene as a target and screening corresponding drugs or preparations, so that the selected drugs or preparations can inhibit the expression of CD300LD gene to realize tumor therapy. For example, small molecule interfering RNA (siRNA) against the human CD300LD gene is selected and can be used as a drug to inhibit tumor cell proliferation. In addition, antibody drugs and small molecule drugs also target the CD300LD gene.

The use of CD300LD gene as a target in the preparation of tumor diagnostic drugs specifically refers to: using the CD300LD gene expression products as tumor diagnostic indicators for the preparation of tumor diagnostic drugs.

In one embodiment, the tumor therapeutic drug is a molecule capable of specifically inhibiting the transcription or translation of the CD300LD gene or specifically inhibiting the expression or activity of the CD300LD protein, which reduces the expression level of the CD300LD gene in tumor cells or competitively inhibits CD300LD, achieving a significant improvement in the tumor immunosuppressive microenvironment. The significant improvement in the tumor immunosuppressive microenvironment refers to the dramatic decrease of PMN-MDSC, the significant increase of CD8+ T cell infiltration, the significant downregulation of PMN-MDSC migration and function-related signaling pathways, and the significant up-regulation of T cell migration and killing-related signaling pathways. The inventors found that CD300LD in the tumor therapeutic drugs regulates the tumor migration and T cell inhibition functions of PMN-MDSC through the downstream S100A8/A9, producing a broad-spectrum anti-tumor effect.

The tumor therapeutic drugs or tumor diagnostic drugs prepared based on the CD300LD gene include, but are not limited to, nucleic acid molecules, carbohydrates, lipids, small molecule chemicals, antibody drugs, polypeptides, proteins, or interfering lentiviruses.

The nucleic acid molecules include, but are not limited to, antisense oligonucleotides, double-stranded RNA (dsRNA), nuclease, small interfering RNA prepared by endoribonuclease III, or short hairpin RNA (shRNA).

In one embodiment, the tumor therapeutic drugs are administered in a dose sufficient to reduce transcription or translation of the human CD300LD gene or to reduce the expression or activity of the CD300LD protein. For example, the expression or activity of the human CD300LD gene is reduced by at least 50%, 80%, 90%, 95%, or 99%.

In another embodiment, the dose sufficient to reduce the activity of the CD300LD protein is an amount sufficient to competitively bind to the CD300LD protein or a receptor thereof, such that the CD300LD protein is unable to function.

A method for tumor treatment using the aforementioned tumor therapeutic drugs is mainly to inhibit tumor cell proliferation by reducing the expression or activity level of the human CD300LD gene. Specifically, a substance that can effectively reduce the expression or activity level of the human CD300LD gene is administered to the patient for treatment.

In one embodiment, the target of the CD300LD gene is Exon2.

The present disclosure also provides a use of CD300LD gene and PD1 together as targets in the preparation of tumor preventive, diagnostic, or therapeutic drugs.

The present disclosure also provides a use of a CD300LD inhibitor in the preparation of products having at least one of the following effects:
tumor prevention, diagnosis, or treatment;
regulation of myeloid cell activity and/or migration;
regulation of T cell activity; and
regulation of CD300LD downstream signaling pathway.

The inventors of the present disclosure have found that CD300LD inhibitors can regulate the activity of myeloid cells. The regulation of myeloid cell activity may refer to, for example, decreasing the number of myeloid cells or the expression of genes related to suppression of immunity on myeloid cells.

The regulation of myeloid cell migration may refer to significantly down-regulating myeloid cell migration-related signaling pathways.

The myeloid cells are selected from macrophages, M-MDSC, and PMN-MDSC.

The regulation of T cell activity may refer to, for example, significantly increasing the number of infiltrating immune cells in the tumor microenvironment or the number of CD4⁺ and CD8⁺ T cells, significantly decreasing the number of Treg cells, or significantly increasing the expression of genes related to the killing function of the T cells or the expression of genes related to the recruitment of T cells. CD300LD inhibitors can regulate T cell proliferation by inhibiting the T cell suppressive function of myeloid cells.

The regulation of downstream signaling pathways of CD300LD may refer to regulating a certain gene in the downstream signaling pathways positively or negatively. The certain gene in the downstream signaling pathways may be, for example, S100A8, S100A9, Trim10, Ms4a6d, and Apol11b.

In the products provided by the present disclosure, the CD300LD inhibitors are typically used as immunotherapeutic drugs. Immunotherapeutic drugs for tumor treatment usually alter the tumor microenvironment by restarting and maintaining the tumor-immune cycle and restoring the body's normal anti-tumor immune response, so as to control and clear the tumor.

The products necessarily comprise the CD300LD inhibitors, and employ the CD300LD inhibitors as the active ingredient for obtaining the foregoing efficacy.

In the products, the active ingredient that performs the aforementioned functions may comprise only the CD300LD inhibitors, or may include other molecules that can perform the aforementioned functions.

That is, the CD300LD inhibitor is the only active ingredient or one of the active ingredients of the product.

The product may be made of a single component or multiple components.

Without any special restrictions, the product may be in the form of a solid, liquid, gel, semi-liquid, aerosol, etc.

The product is mainly administered to mammals. The mammals are preferably rodents, even-toed ungulates, odd-toed ungulates, lagomorphs, primates, and the like. The primates are preferably monkeys, apes, or humans.

The products include, but are not limited to, drugs, healthcare products, foodstuffs, kits, and the like.

The CD300LD inhibitor may be a nucleic acid molecule, an antibody, or a small molecule compound.

As listed in embodiments of the present disclosure, the CD300LD inhibitor may be a nucleic acid molecule that reduces the expression of the CD300LD gene in tumor cells.

In the present disclosure, the CD300LD inhibitor typically refers to a substance that can inhibit the expression and/or function of CD300LD. For example, the CD300LD inhibitor may partially inhibit, i.e., reduce the expression and/or function of CD300LD, e.g. reduce the expression and/or function of CD300LD by 50%, 60%, 70%, 80%, 90%, 95%, 99%, or may completely inhibit, i.e., essentially completely eliminate the expression and/or function of CD300LD. Suitable kinds of substances capable of acting as CD300LD inhibitors are known to those skilled in the art. For example, the CD300LD inhibitor may be an antagonist, a blocker, and the like. For further example, the inhibitory function of the CD300LD inhibitor is an expression level inhibition at the level of nucleic acid molecules (e.g., at the level of mRNA, at the level of DNA) and/or at the level of protein molecules of the CD300LD gene. As a further example, the CD300LD inhibitor may also be a substance that competitively binds to ligands of CD300LD or a substance that competitively binds to CD300LD. More specifically, the CD300LD inhibitor may be a nucleic acid molecule, a protein molecule, or a compound. For example, the nucleic acid molecule may be selected from an interfering RNA targeting CD300LD, an antisense oligonucleotide targeting CD300LD, a substance for knocking out or knocking down CD300LD, and more specifically may be siRNA, miRNA, shRNA, gene knock-out vectors, gene expression vectors (e.g., capable of expressing siRNA, shRNA, interfering RNA), and the like. In a specific embodiment of the present disclosure, the target sequence of the nucleic acid molecule may be exon 2 of CD300LD. For example, the protein molecule may be selected from anti-CD300LD antibodies, which may be monoclonal antibodies, polyclonal antibodies, and the like. As another example, the CD300LD inhibitor may be CD300LD-ECD (CD300LD extracellular region), and the amino acid sequence of CD300LD-ECD can be derived from rats, mice, humans, and the like.

The present disclosure provides an isolated polypeptide, where the isolated polypeptide comprises a functional fragment of a CD300LD protein, and the functional fragment of the CD300LD protein is the extracellular region of the CD300LD protein.

The functional fragment is capable of binding to a ligand of the CD300LD protein.

In one embodiment, the extracellular region of the CD300LD protein is derived from a mammalian CD300LD protein or a mutant thereof. The mammals are preferably rodents, even-toed ungulates, odd-toed ungulates, lagomorphs, primates, and the like. The rodents are preferably rats. The primates are preferably monkeys, apes, or humans.

In one embodiment, the amino acid sequence of the extracellular region of CD300LD is shown as SEQ ID NO. 17 or SEQ ID NO. 31. In some embodiments, the extracellular region of CD300LD is selected from one or more fragments of the amino acid sequence shown as SEQ ID NO.17 or SEQ ID NO.31.

In some embodiments of the present disclosure, a mutant of the mammalian CD300LD protein is a polypeptide having substitution, deletion, or addition of one or more amino acids in the aforementioned amino acid sequence and possesses equivalent activity. The mutant may have at least 80%, 85%, 90%, 93%, 95%, 97%, 99%, or more similarity to the foregoing amino acid sequence.

The isolated polypeptide further comprises other peptide fragments translated by an expression vector expressing the polypeptide.

Specifically, the isolated polypeptide further comprises a protein purification tag.

The protein purification tag is used to isolate and purify the expressed protein. There are many protein purification tags and they are commonly used in the art. Non-limiting examples of suitable protein purification tags include fluorescent proteins, Fc tags, His tags, poly(His) tags, FLAG (or DDK) tags, Halo tags, AcV5 tags, AU1 tags, AU5 tags, biotin carboxyl carrier proteins (BCCPs), calmodulin binding proteins (CBPs), chitin-binding structural domains (CBDs), E tags, E2 tags, ECS tags, eXact tags, Glu-Glu tags, glutathione-S-transferase (GST), HA tags, HSV tags, KT3 tags, maltose-binding proteins (MBPs), MAP tags, Myc tags, NE tags, NusA tags, PDZ tags, S tags, S1 tags, SBP tags, Softag 1 tags, Softag 3 tags, Spot tags, Strep tags, SUMO tags, T7 tags, tandem affinity purification (TAP) tags, V5 tags, VSV-G tags, and Xa tags. Non-limiting examples of suitable fluorescent proteins include green fluorescent proteins (e.g., GFP, GFP-2, tagGFP, turboGFP, EGFP, Emerald, Azami Green, monomeric Azami Green, CopGFP, AceGFP, ZsGreen1), yellow fluorescent proteins (e.g., YFP, EYFP, Citrine, Venus, YPet, PhiYFP, ZsYellow1), blue fluorescent proteins (e.g., EBFP, EBFP2, Azurite, mKalama1, GFPuv, Sapphire, T-sapphire), cyan fluorescent proteins (e.g. ECFP, Cerulean, CyPet, AmCyanl, Midoriishi-Cyan), red fluorescent proteins (e.g., mKate, mKate2, mPlum, DsRed monomer, mCherry, mRFP1, DsRed-Express, DsRed2, DsRed monomer, HcRed-Tandem, HcRed1, AsRed2, eqFP611, mRasberry, mStrawberry, Jred), orange fluorescent proteins (e.g., mOrange, mKO, Kusabira-Orange, monomeric Kusabira-Orange, mTangerine, tdTomato) and any other suitable fluorescent proteins.

The function of the extracellular region of the CD300LD protein is not affected by the protein purification tag.

The inventor's research results show that CD300LD is a novel tumor immunosuppressive receptor specific for polymorphonuclear myeloid-derived suppressor cells (PMN-MDSC), where PMN-MDSC is a class of pathologically induced neutrophils widely present in various tumors, which suppresses the functions of effector cells, such as T-cells and NK cells, and promotes tumor development, infiltration, and metastasis, thus playing a key role in tumor immunomodulation. The isolated peptide of the present disclosure utilizes the competitive inhibition mechanism of CD300LD-ECD protein against CD300LD to suppress tumor development. The isolated peptide, when administered to tumor-bearing mice, significantly inhibits tumor development. Additionally, CD300LD-ECD, when used in combination with PD1 antibodies, exhibits a significant synergistic anti-tumor effect.

Another aspect of the present disclosure provides an isolated polynucleotide, where the isolated polynucleotide encodes the isolated polypeptide.

In one embodiment, the sequence of the isolated polynucleotide is shown as SEQ ID NO. 16 or SEQ ID NO. 30.

In some embodiments of the present disclosure, the isolated polynucleotide may be a polynucleotide having substitution, deletion, or addition of one or more nucleotides in the aforementioned sequence and possesses equivalent activity. The polynucleotide sequence with substitution, deletion, or addition may have 80%, 85%, 90%, 93%, 95%, 97%, 99%, or more similarity to the aforementioned polynucleotide.

In certain embodiments of the present disclosure, the amino acid sequence of the isolated polypeptide is shown as SEQ ID NO. 33 or SEQ ID NO. 34.

Another aspect of the present disclosure provides a nucleic acid construct comprising the isolated polynucleotide.

In certain embodiments of the present disclosure, the nucleic acid construct is formed by inserting the isolated polynucleotide into a polyclonal site of an expression vector. Expression vectors in the present disclosure generally refer to a variety of commercially available expression vectors known in the art, which may be, for example, bacterial plasmids, phages, yeast plasmids, plant cellular viruses, mammalian cellular viruses such as adenoviruses and retroviruses, or other vectors.

In certain embodiments of the present disclosure, the expression vector is selected from pCMV-hFc expression vectors.

In certain embodiments of the present disclosure, the nucleic acid construct has a nucleotide sequence shown as SEQ ID NO. 19 or SEQ ID NO. 32.

Another aspect of the present disclosure provides a cell line, where the cell line comprises the nucleic acid construct, or a genome of the cell line incorporates an exogenous polynucleotide as desribed above.

In certain embodiments of the present disclosure, the cell line is obtained by transfecting the nucleic acid construct into a host cell. Any cell suitable for expression of an expression vector can serve as a host cell, for example, the host cell may be a prokaryotic cell, such as a bacterial cell; a lower eukaryotic cell, such as a yeast cell; or a higher eukaryotic cell, such as a mammalian cell. In one embodiment, the host cell is a 293e cell.

The present disclosure also provides a method for preparing the isolated polypeptide, where the method comprises the following steps:
a) culturing a cell line as described above under expression conditions to express the isolated polypeptide;
b) isolating and purifying the polypeptide described in step a).

The present disclosure provides a use of the isolated polypeptide in the preparation of a product for tumor prevention, diagnosis, or treatment.

In the present disclosure, the term "prevention/preventive" includes prophylactic interventions leading to a desired pharmacological and/or physiological effect. Preferably, the effect is a medical ability to inhibit or delay the onset of a disease, and/or to reduce the risk of disease development or deterioration.

In the present disclosure, the term "diagnosis/diagnostic" refers to determining a disease based on the expression of CD300LD.

In the present disclosure, the term "treatment/therapeutic" includes curative or palliative interventions leading to a desired pharmacological and/or physiological effect. The effect is preferably a medical ability to reduce one or more symptoms of a disease or completely eliminate the disease.

The tumor is a CD300LD overexpressing tumor. The CD300LD overexpression typically means that the CD300LD expression level is above a certain threshold, and this expression can be at the molecular level or the protein level. For example, CD300LD positive may be the overexpression of CD300LD mRNA in tumor tissue. For another example, CD300LD positive may be the overexpression of CD300LD protein in the tumor tissue. For example, CD300LD positive may be that the tumor tissue has a higher expression level of CD300LD mRNA than the surrounding healthy tissue. For example, CD300LD positive may be that tumor tissue has a higher expression level of CD300LD protein than the surrounding healthy tissue. The tumor may be a solid tumor or a blood tumor, and more specifically may be an intestinal cancer, a lung cancer, a liver cancer, a breast cancer, an esophageal cancer, a head and neck cancer, a skin cancer, a kidney cancer, a leukemia, a cervical cancer, a coad (colon cancer), a lihc (liver hepatocellular carcinoma), an ov (ovarian serous carcinoma), an ucec (uterine corpus endometrial carcinoma), a thca (thyroid cancer), a skcm (skin cutaneous melanoma), a luad (lung adenocarcinoma), a hnsc (head and neck squamous cell carcinoma), a gbm (glioblastoma multiforme), a prad (prostate adenocarcinoma), a thym (thymic carcinoma), a lgg (brain low-grade glioma), a read (rectal adenocarcinoma), a pcpg (pheochromocytoma and paraganglioma), an esca (esophageal carcinoma), a kirc (kidney renal clear cell carcinoma), a blca (bladder urothelial carcinoma), a kirp (kidney renal papillary cell carcinoma), a paad (pancreatic adenocarcinoma), a stad (stomach adenocarcinoma), a kich (chromophobe renal cell carcinoma), a brca (breast invasive carcinoma), a lusc (lung squamous cancer), a sarc (sarcoma), a LAMI, (acute myeloid leukemia), and others.

The product necessarily comprises the isolated polypeptide, where the isolated polypeptide is the sole active ingredient or one of the active ingredients.

In certain embodiments, the present disclosure provides a use of the isolated polypeptide and the anti-PD1 antibody in the preparation of a product for tumor prevention, diagnosis, or treatment.

The anti-PD1 antibody may be any antibody targeting PD1, such as Opdivo, Keytruda, or BioXcell's InVivoMab anti-mouse PD-1 (CD279), Cat#BE0273.

The product may be made of a single component or multiple components.

Without any special restrictions, the product may be in the form of a solid, liquid, gel, semi-liquid, aerosol, etc.

The product is mainly administered to mammals. The mammals are preferably rodents, even-toed ungulates, odd-toed ungulates, lagomorphs, primates, and the like. The primates are preferably monkeys, apes, or humans. The rodents are preferably rats.

The products include, but are not limited to, drugs, healthcare products, foodstuffs, kits, and the like.

The product can competitively bind to ligands of CD300LD. Since it only includes the extracellular region of CD300LD, excluding the transmembrane and intracellular regions, signal transmission to the intracellular region is inhibited after the extracellular region binds to the ligand. This simulates CD300LD knockout, effectively inhibiting or completely eliminating the function of CD300LD, and ultimately facilitating tumor immunotherapy.

The present disclosure also provides a composition, where the composition comprises the CD300LD inhibitor and has at least one of the following effects:
tumor treatment;
regulation of myeloid cell activity and/or migration;
regulation of T cell activity; and
regulation of CD300LD downstream signaling pathways.

The CD300LD inhibitor may be any CD300LD inhibitor as described above.

In the present disclosure, the product or composition may be used for diagnostic or therapeutic purposes, or may be used for non-diagnostic or non-therapeutic purposes.

In one embodiment, the composition is a pharmaceutical composition, where the pharmaceutical composition comprises one or more pharmaceutically acceptable carriers or excipients, and the CD300LD inhibitor is the isolated polypeptide.

"Pharmaceutically acceptable" means that when the molecular entity and the composition are appropriately administered to an animal or human, they do not produce adverse, allergic, or other undesirable reactions.

A "pharmaceutically acceptable carrier or excipient" should be compatible with the active ingredient as described, meaning it can be mixed with the active ingredient without significantly reducing the effectiveness of the drug under normal circumstances. Specific examples of substances that can be used as pharmaceutically acceptable carriers or excipients include sugars, such as lactose, glucose, and sucrose; starches, such as corn starch and potato starch; cellulose and its derivatives, such as sodium methylcellulose, ethylcellulose, and methylcellulose; Tragacanth powder; malt; gelatin; talc; solid lubricants, such as stearic acid and magnesium stearate; calcium sulfate; vegetable oils, such as peanut, cottonseed, sesame, olive, corn, and cocoa oils; polyols, such as propylene glycol, glycerol, sorbitol, mannitol, and polyethylene glycol; alginic acid; emulsifiers, such as Tween; wetting agents, such as sodium lauryl sulfate; coloring agents; flavoring agents; tableting agents, stabilizers; antioxidants; preservatives; pyrogen-free water; isotonic salt solutions; and phosphate buffers. These substances are utilized, as necessary, to enhance the stability of the formulation, improve activity or bioavailability, or impart an acceptable taste or odor particularly in the case of oral administration.

In the present disclosure, unless otherwise indicated, pharmaceutical dosage forms are not specifically limited, and may be made into injections, oral liquids, tablets, lyophilized powders, capsules, drops, sprays, and other dosage forms that can be prepared by conventional methods. The drug dosage form matches the administration mode.

The present disclosure also provides a method for in vitro screening of a drug, where the method comprises the following steps: mixing CD300LD-overexpressed cells with a drug to be screened and monitoring the changes in cells.

In one embodiment, the drug to be screened is an anti-tumor drug. The anti-tumor drug is, for example, the CD300LD inhibitor.

In one embodiment, the CD300LD-overexpressed cells are myeloid immune cells. More specifically, the myeloid immune cells are selected from macrophages, M-MDSC, and PMN-MDSC.

The changes in cells include changes in cell activity, changes in cell migration, changes in CD300LD expression, changes in CD300LD downstream signaling pathway, and the like.

The present disclosure also provides a method for regulation, the method comprises administering to an individual an effective amount of the CD300LD inhibitor or the composition as provided hereinbefore to regulate the myeloid cell activity, T cell activity, or CD300LD downstream signaling pathway.

The present disclosure also provides a method for treating CD300LD overexpression diseases, the method comprises administering to an individual a therapeutically effective amount of the isolated polypeptide or the composition as provided hereinbefore.

The CD300LD overexpression diseases described herein include, but are not limited to, tumors.

In the present disclosure, "individual" typically includes human, and non-human primates such as mammals, dogs, cats, horses, sheep, pigs, cows, etc., which may benefit from treatment with the drug, the pharmaceutical composition, the preparation, the kit, or the combined preparation as described above.

In the present disclosure, "therapeutically effective amount" generally refers to an amount that, after a suitable period of administration, can achieve a therapeutic effect for the treatment of the aforementioned diseases. Specifically, when the isolated polypeptide or pharmaceutical composition is administered to a subject, the dosage varies depending on the age and weight of the patient, the characteristics and severity of the disease, and the route of administration, with reference to the results of animal experiments under various circumstances, and the total dosage should be within a certain range.

In some embodiments, the isolated polypeptide or pharmaceutical composition described herein is administered in an amount of about 0.001 mg/Kg to about 500 mg/Kg (e.g., about 0.001 mg/Kg to about 200 mg/Kg; about 0.01 mg/Kg to about 200 mg/Kg; about 0.01 mg/Kg to about 150 mg/Kg; about 0.01 mg/Kg to about 100 mg/Kg; about 0.01 mg/Kg to about 50 mg/Kg; about 0.01 mg/Kg to about 10 mg/Kg; about 0.01 mg/Kg to about 5 mg/Kg; about 0.01 mg/Kg to about 1 mg/Kg; about 0.01 mg/Kg to about 0.5 mg/Kg; about 0.01 mg/Kg to about 0.1 mg/Kg; about 0.01 mg/Kg to about 200 mg/Kg; about 0.1 mg/Kg to about 150 mg/Kg; about 0.1 mg/Kg to about 100 mg/Kg; about 0.1mg/Kg to about 50 mg/Kg; about 0.1mg/Kg to about 10mg/Kg; about 0.1mg/Kg to about 5 mg/Kg; about 0.1mg/Kg to about 1 mg/Kg; about 0.1mg/Kg to about 0.5 mg/Kg). The total daily dose may be divided for administration and given at intervals throughout the day by continuous delivery. The drug may be administered daily (e.g., as a single dose or as two or more doses) or non-daily (e.g., every other day, every two days, every three days, once a week, twice a week, once every two weeks, once a month). In some embodiments, the isolated polypeptide or pharmaceutical composition described herein is administered for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or longer. In another embodiment, the discontinuation of administration lasts for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or longer. In one embodiment, the individual is administered with the therapeutically isolated peptide or pharmaceutical composition for some time, followed by a period without administration. In another embodiment, the therapeutically isolated polypeptide or pharmaceutical composition is administered for a first period. The administration is then discontinued for a second period, followed by restarting the administration of the isolated polypeptide or pharmaceutical composition for a third period. Subsequently, the administration is discontinued for a fourth period after the third period. In one embodiment, the administration lasts for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months or longer. In another embodiment, the discontinuation of administration lasts for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or longer.

In some embodiments, the methods described herein can further comprise administering one or more additional therapies (e.g., one or more additional therapeutic agents and/or one or more treatment regimens) in combination with the isolated polypeptide described herein.

In some embodiments, the methods described herein can further comprise administering one or more additional cancer therapies. The one or more additional cancer therapies may include, but are not limited to: surgery, radiation therapy, chemotherapy, toxinotherapy, immunotherapy, cryotherapy, cancer vaccines (e.g., HPV vaccine, Hepatitis B vaccine, Oncophage, Provenge), and gene therapy, and combinations thereof. Immunotherapies include, but are not limited to, adoptive cell therapy, stem cell and/or dendritic cell derivation, blood transfusion, lavage, and/or other therapies, including, but not limited to, freezing tumors.

In some embodiments, the one or more additional cancer therapies are chemotherapies, which include administering one or more additional chemotherapeutic agents.

In some embodiments, the additional chemotherapeutic agents are immunomodulatory molecules, such as immune checkpoint inhibitors. In some of these embodiments, the immune checkpoint inhibitors target an immune checkpoint receptor selected from: CTLA-4, PD-1, PD-L1, PD-1-PD-L1, and PD-1-PD-L2.

In some of these embodiments, the immune checkpoint inhibitors are selected from: Pembrolizumab (PD1), Nivolumab (PD1), Atezolizumab (formerly known as MPDL3280A) (PDL1), MEDI4736 (PD-L1), Avelumab (PD-L1), PDR001 (PD1), BMS-986016, MGA271, Lirilumab, IPH2201, Emactuzumab, INCB024360, Galunisertib, Ulocuplumab, BKT140, Bavituximab, CC-90002, Bevacizumab, MNRP1685A, and MGA271.

Embodiments of the present disclosure are described below by specific examples, and other advantages and efficacies of the present disclosure can be readily appreciated by those skilled in the art from the contents herein. The present disclosure may also be implemented or applied in various other specific embodiments, and various details in this specification may be modified or altered based on different viewpoints and applications without departing from the spirit of the present disclosure.

Before further describing the specific embodiments of the present disclosure, it should be understood that the protection scope of the present disclosure is not limited to the particular specific embodiments described below. It should also be understood that the terms used in the embodiments of the present disclosure are intended to describe the particular embodiments and are not intended to limit the protection scope of the present disclosure. In the specification and claims of the present disclosure, unless otherwise expressly stated in the text, the singular forms "a", "one", and "the" also include the plural form.

When numerical ranges are given in the embodiments, it should be understood that, unless otherwise specified in the present disclosure, the two endpoints of each numerical range and any value between the two endpoints can be selected. Unless otherwise defined, all technical and scientific terms used in the present disclosure have the same meaning as commonly understood by those skilled in the art. In addition to the specific methods, devices, and materials used in the embodiments, based on the understanding of those skilled in the art and the disclosure of the present disclosure, any methods, devices, and materials similar or equivalent to those described in the embodiments can be used to implement the present disclosure.

### Embodiment 1 Screening key functional receptors of PMN-MDSC

The immunosuppressive microenvironment of a tumor is an important factor in tumor immune escape. In the tumor microenvironment, polymorphonuclear myeloid-derived suppressor cells (PMN-MDSC) are an important cell population leading to immunosuppression. Since PMN-MDSC and neutrophils are similar in molecular typing but opposite in function, in this embodiment, transcriptome sequencing is preformed on PMN-MDSC and neutrophils to analyze the differences in membrane protein expressions, meanwhile, a sgRNA library is constructed for PMN-MDSC membrane proteins, and an in vivo screening using CRISPR-Cas9 is performed in combination with a mouse tumor model. The key functional receptors of PMN-MDSC can be screened using these strategies, and the obtained candidate molecules are analyzed by expression profile.

### Materials and Methods:

1-1) Transcriptome analysis of PMN-MDSC and neutrophils: neutrophils (CD11b⁺Ly6G⁺) in the bone marrow of normal C57 mice and PMN-MDSC (CD11b⁺Ly6G⁺) from the spleens of B16-F10 tumor-bearing mice were isolated by using AutoMACS magnetic sorting, and total cellular RNA was extracted according to methods well known in the field and analyzed by RNAseq sequencing.

1-2) Construction and packaging of sgRNA libraries targeting surface receptors specific to myeloid cells: the expression ratios of membrane protein genes in myeloid cells/lymphoid cells were calculated according to RNAseq data from immune cells, and genes with a ratio >2 were selected, resulting in 300 receptor genes specifically expressed in myeloid cells. For each gene, 10 sgRNA oligos were designed, synthesized, and constructed into Syn003-modified lentiviral expression plasmids using well-known methods in the field, creating an sgRNA lentiviral expression library targeting receptors on the surface of myeloid cells. The library plasmids were co-transfected with lentiviral packaging plasmids into 293T cells using Lipofectin. After 48 hours, the viral supernatant was collected, filtered through a 0.45µm filter membrane, and used for infecting target cells.

1-3) Screening of myeloid cell-related key receptors in the tumor microenvironment: hematopoietic precursor cells (lineage-negative cells) were isolated from the fetal liver of Cas9 mice using magnetic sorting. These precursor cells were then infected with the aforementioned sgRNA library virus supernatant and transplanted into irradiated mice (9G) to reconstitute the mouse immune system. The immune cells of these mice will express different sgRNAs.Four weeks after transplantation, 1.5×10⁵ B16 cells were subcutaneously inoculated into the mice to induce tumor formation. Two weeks later, myeloid cells from the mouse bone marrow and tumor were isolated, and gRNAs were amplified and subjected to deep sequencing and comparative analysis using well-known methods in the field.

1-4) Analysis of CD300LD expression in various types of immune cells in mice: Bone marrow of normal mice, as well as bone marrow and tumors of B16 tumor-bearing mice were isolated, prepared into single-cell suspensions, labeled with antibodies against CD300LD and antibodies against each immune cell, and then subjected to flow-through assay to analyze the protein expression level of CD300LD on the surface of each type of immune cell, where the surface markers of the immune cells were: macrophages (CD45⁺/CD11b⁺//F4/80⁺), monocytes/M-MDSC (CD45⁺/CD11b⁺/Ly6c⁺), neutrophils/PMN-MDSC (CD45⁺/CD11b⁺/Ly6G⁺), DCs (CD45⁺/CD11b⁻/CD11c⁺), T cells (CD45⁺/CD3⁺), CD4⁺T (CD45⁺/CD3⁺/CD4⁺), CD8⁺T (CD45⁺/CD3⁺/CD8⁺), B cells (CD45⁺/B220⁺), and NK cells (CD45⁺/NKG2D⁺). Immune cells from bone marrow in normal mice as well as bone marrow and tumor in tumor-bearing mice were sorted by the above molecular markers to analyze the RNA expression level of CD300LD, and mRNAs were extracted, reversely transcribed by oligo d(T)16 (Invitrogen, Cat#N8080128), and then subjected to qRT-PCR with the following primers and conditions.
mLD-For: 5' ACATTGACCAAGCCCCAAAAA3' (SEQ ID NO.1)
mLD-Rev: 5'AGCAGGGGGTAACTCCACAAAG 3' (SEQ ID NO.2)
mGAPDH-For: 5'GTCTACATGTTCCAGTATGACTCC 3' (SEQ ID NO.3)
mGDPDH-Rev: 5'AGTGAGTTGTCATATTTCTCGTGGGT 3' (SEQ ID NO.4)
qPCR conditions: 95°C for 5min, (95°C for 30s, 58°C for 30s, 72°C for 30s, 40cycles), 72°C for 10min, and 4°C for 10min.

1-5) Analysis of CD300LD expression in various types of human immune cells: the mRNA expression level of CD300LD in different types of human immune cells was analyzed by using the Human Protein Atlas database. Peripheral blood from normal people and intestinal cancer patients was collected, and mRNAs of leukocytes were extracted and then subjected to qRT-PCR detection according to the following primers and conditions, to analyze the expression level of CD300LD in clinical oncology patients.
huLD-For: 5'TCCCAGGTTACTCCATTGCC 3' (SEQ ID NO.5)
huLD-Rev: 5'GCCTGAGCCATAAGCACACT 3' (SEQ ID NO.6)
huGAPDH-For: 5'TGTGGGCATCAATGGGATTTGG 3' (SEQ ID NO.7)
huGDPDH-Rev: 5'ACACACCATGTATTCCGGGGTCAAT 3' (SEQ ID NO. 8)
qPCR conditions: 95°C for 5min, (95°C for 30s, 58°C for 30s, 72°C for 30s, 40cycles), 72°C for 10min, and 4°C for 10min).

### Results:

Transcriptome analysis showed that CD300LD was significantly upregulated in PMN-MDSC compared to normal neutrophils (FIG. 1A). Meanwhile, an in vivo screening of membrane proteins specifically expressed in myeloid cells using Crispr-Cas9 (FIG. 1B) showed that the abundance of CD300LD-gRNA in myeloid cells in tumor microenvironment was significantly lower than that in myeloid cells in bone marrow for tumor-bearing mice (FIG. 1C), suggesting that the tumor microenvironment rejected the knockout of CD300LD. Both of these screens suggested that CD300LD may be a key receptor for PMN-MDSC.

CD300LD-expressing cells were analyzed. CD300LD was specifically highly expressed in normal neutrophils, as well as in tumor PMN-MDSC (FIGs. ID and E). CD300LD expression levels were significantly higher in PMN-MDSC compared to normal neutrophils (FIG. 1F). In human immune cell populations, CD300LD was also specifically highly expressed on neutrophils (FIG. 1G); and the expression level of CD300LD PMN-MDSC derived from tumor patients was significantly higher than that in normal neutrophils (FIG. 1H).

The above results indicated that CD300LD was specifically highly expressed on neutrophils/PMN-MDSC, and its expression on PMN-MDSC cells was significantly elevated during tumorigenesis; combined with the results of Crispr-Cas9 screening, which indicated that the tumor microenvironment rejected CD300LD knockout, it suggested that CD300LD was likely to be a key receptor required for PMN-MDSC recruitment, differentiation, and function in tumors.

Embodiment 2 CD300LD knockout significantly inhibits the development of various types of tumors

To assess the function of CD300LD in tumor development, CD300LD-knockout mice were constructed, and various different tumor models were used to analyze the effect of CD300LD knockout on tumor development.

### Materials and Methods:

2-1) Construction of CD300LD knockout mice (entrusted to a third-party company, GemPharmatech): CD300LD Exon2-knockout (nucleotide sequence was shown as SEQ ID NO.11) mice were constructed using Cas9/sgRNA (the sequence of gRNA1 was shown as SEQ ID NO.9; the sequence of gRNA2 was shown as SEQ ID NO.10).

2-2) B16-F10 melanoma tumor mouse model: B16 cells (1.5×10⁵ cells/mouse) were inoculated into mice subcutaneously, the size of the tumor was measured every 2-3 days, the tumor growth curves were plotted, the mice with a tumor volume of 1000cm³ were deemed dead, and the survival curves of mice were plotted.

2-3) TC1 cervical cancer model: TC1 cells (1.5×10⁵ cells/mouse) were inoculated into mice subcutaneously, the tumor size was measured every 2-3 days, the tumor growth curve was plotted, the mice with a tumor volume of 1000cm³ were deemed dead, and the survival curve of mice was plotted.

2-4) MC-38 colon cancer model: MC38 cells (1.0×10⁶ cells/mouse) were inoculated into mice subcutaneously, the size of the tumor was measured every 2-3 days, the tumor growth curve was plotted, the mice with a tumor volume of 1000cm³ were deemed dead, and the survival curve of mice was plotted.

2-5) LLC lung cancer model: LLC cells (5×10⁵ cells/mouse) were inoculated into the mice subcutaneously, the size of the tumor was measured every 2-3 days, the tumor growth curve was plotted, the mice with a tumor volume of 1000cm³ were deemed dead, and the survival curve of mice was plotted.

### Results:

CD300LD knockout mice were constructed, and CD300LD was detected to be knocked out at the genomic level and protein level (FIGs. 2A-C). Tumor growth in CD300LD knockout mice was found to be significantly slower than that in wild-type mice under the B16F10 melanoma tumor model (FIGs. 2D and E), and the survival time of CD300LD knockout mice was also significantly prolonged (FIG. 2F). Tumor growth in CD300LD-knockout mice was also significantly lower than that in wild-type mice under the TC1 cervical cancer model, MC38 colon cancer model, and LLC lung cancer model (FIGs. 2G-I). The above results demonstrated that knockout of CD300LD in hosts significantly inhibited the development of multiple types of tumors.

### Embodiment 3 CD300LD knockout inhibits tumor development via PMN-MDSC

CD300LD-knockout mice were able to significantly inhibit the development of various types of tumors, therefore, it is extremely critical to determine which cell population's CD300LD plays a role. CD300LD is mainly expressed on myeloid immune cells, including macrophages, M-MDSC, and PMN-MDSC, with the expression level of CD300LD on PMN-MDSC being much higher than that on other cell types (FIGs. 1D and E). In this embodiment, it was determined which type of myeloid cells with their CD300LD knocked out inhibited tumor development.

### Materials and Methods:

3-1) Effect of conditional knockout of CD300LD in different cell populations on tumor development: CD300LD fl/fl conditional knockout mice were constructed (entrusted to a third-party company, GemPharmatec; the sequence of gRNA1 was shown as SEQ ID NO.12 and the sequence of gRNA2 was shown as SEQ ID NO.13), and the mice were mated with different Cre mice, including LyzCre (Cre was specifically expressed in myeloid cells such as macrophages, neutrophils/PMN-MDSC, etc.) and S100A8Cre (Cre was specifically expressed in neutrophil/PMN-MDSC), so as to obtain mice with CD300LD conditional knockout in different types of myeloid cells. These mice were subjected to a B16-F10 tumor experiment to detect tumor growth (see step 2-2 in Embodiment 2).

3-2) Effect of PMN-MDSC removal by Ly6G antibody on tumor development: B16-F10 tumor cells were subcutaneously inoculated into mice, Ly6G antibody or Isotype control antibody (200 µg/mouse) was injected on days 7, 9, 11, and 13 after inoculation, and tumor growth was detected.

3-3) Effect of co-transplantation of different myeloid cells and tumor cells on tumor growth: macrophages, M-MDSC, and PMN-MDSC cells were isolated from WT or CD300LD-KO tumor-bearing mice, mixed with B16-F10 cells at a ratio of 1:1, then inoculated into wild-type mice subcutaneously for a tumorigenic assay, and the growth of tumors was detected.

### Results:

The results showed that conditional knockout of CD300LD using LyzCre in a variety of myeloid cells (including macrophages/neutrophils/PMN-MDSC) significantly inhibited the development of B16 tumor (FIG. 3A), and conditional knockout of CD300LD using the more specific S100A8Cre in PMN-MDSC likewise significantly inhibited the development of the tumor (FIG. 3B), indicating that CD300LD expressed by PMN-MDSC plays an important role in tumor development.

To further demonstrate that CD300LD inhibits tumors via PMN-MDSC, Ly6G antibody was used to remove neutrophils/PMN-MDSC in wild-type tumor-bearing mice and CD300LD-KO tumor-bearing mice, the results showed a significant slowing of tumor development in wild-type mice, whereas Ly6G antibody was ineffective in CD300LD-KO mice (FIG. 3C), indicating that the antitumor effects of CD300LD knockout were mainly mediated by neutrophils/PMN-MDSC.

To further demonstrate that CD300LD regulates tumor development through PMN-MDSC, macrophages, M-MDSC, and PMN-MDSC cells were isolated from CD300LD WT and CD300LD KO tumor-bearing mice, and then the effects of different cell populations on tumor development were analyzed using co-injection. The results showed that only CD300LD-KO PMN-MDSC significantly inhibited tumor development (FIGs. 3D-F).

According to the above, the results suggested that CD300LD plays an important role in tumor development mainly through PMN-MDSC cells.

### Embodiment 4 CD300LD knockout improves tumor immunosuppressive microenvironment

To study how CD300LD knockout inhibits tumor development, flow analysis and immunohistochemistry were used to analyze changes in each immune cell population in the tumor microenvironment of WT and CD300LD knockout mice, and single-cell sequencing analysis was performed on immune cells in the tumor microenvironment.

### Materials and Methods:

4-1) B16-F10 melanoma tumor mouse model: B16-F10 cells (at 1.5×10⁵ cells/mouse) were inoculated subcutaneously into mice, and the size of tumors was measured every 2-3 days.

4-2) Tumor microenvironment analysis: single-cell suspensions were prepared from mouse tumors and labeled with two sets of antibodies: antibody group 1 (Anti-Ly6C FITC, Anti-MHCII PE, Anti-F4/80 PE-Cy7, Anti-Cd11b PB, Anti-CD45 BV510, Anti-Cd11c APC, Anti-Ly6G, APC-Cy7) and antibody group 2 (Anti-CD3 FITC, Anti-CD8 PE, Anti-B220 PE-Cy7, Anti-CD45 BV510, Anti-CD49b APC, Anti-CD4 APC-Cy7), and the proportions of respective immune cell populations were determined by flow analysis.

4-3) Immunofluorescence: mouse tumors were isolated, sliced under frozen conditions, stained with CD8-FITC antibody or Ly6G-PE antibody at 4°C overnight, and washed 3 times. The slices were mounted onto slides with DAPI-containing mounting medium, and then observed under a fluorescence microscope.

4-4) Single-cell sequencing of the tumor immune microenvironment: single-cell suspensions were prepared from tumor tissues of B16 tumor-bearing mice, CD45+ immune cells were sorted, 10× single-cell sequencing was carried out, and Sereut was used to perform dimensionality reduction and cluster analysis.

### Results:

The results in FIG. 4 showed that compared to the tumor microenvironment of WT mice, CD300LD knockout resulted in a significant increase in infiltrating immune cells and a significant decrease in PMN-MDSC cells in the tumor microenvironment, accompanied by a significant increase in CD4⁺ and CD8⁺ T cells, as well as a significant decrease in Treg cells (FIGs. 4A and B). Immunofluorescence results similarly showed a significant decrease in PMN-MDSC and a significant increase in CD8⁺ T cells in the tumors of CD300LD knockout mice (FIGs. 4C and D).

This embodiment further analyzed the effect of CD300LD knockout on the tumor immune microenvironment at the single-cell transcriptome level. The results likewise showed that CD300LD knockout resulted in a significant reduction in PMN-MDSC and a significant increase in CD8⁺ T cell infiltration in the tumor microenvironment (FIGs. 5A-C). Meanwhile, the expression of several important genes related to PMN-MDSC immunosuppression, such as S100a8, S100a9, Cxcr2, Cxcl2, Cxcl3, etc., was reduced, the expression of several important genes related to the killing function of T cells, such as Gzma, Gzmb, Ifng, Prf1, Klrk1, etc., was increased significantly, and the expression of genes related to T-cell recruitment, such as Cxcl10, Cxcl9, Ccl5 and H2Ab, was also significantly increased (FIG. 5D).

The above results demonstrated that CD300LD knockout resulted in a significant decrease in PMN-MDSC, an immunosuppressive cell population, in the tumor microenvironment, and a significant increase in immune effector T cells, indicating that CD300LD knockout significantly improved the immunosuppressive microenvironment in tumors.

### Embodiment 5 CD300LD knockout inhibits PMN-MDSC migration and function

Since CD300LD knockout inhibits tumor development by regulating PMN-MDSC, and PMN-MDSC drastically decreases in the tumor microenvironment after the knockout, therefore, whether CD300LD knockout affects the migration process of PMN-MDSC was further verified in this embodiment. As an immunosuppressive cell, the significant role of PMN-MDSC is to inhibit the activity of T-cells, therefore, whether CD300LD knockout affects the immunosuppressive function of PMN-MDSC was also analyzed in this Embodiment.

### Materials and Methods:

6-1) RNAseq: spleens in WT and CD300LD-KO B16 tumor-bearing mice were isolated, single-cell suspensions were prepared and labeled with antibodies, PMN-MDSC cells (CD45+/CD1 lb+/Ly6G+) were sorted, and total cellular RNAs were extracted and reversely transcripted to cDNAs followed by transcriptome sequencing and analysis.

6-2) PMN-MDSC in vitro cell migration assay (Transwell): single-cell suspensions were prepared from isolated spleens of tumor-bearing mice and labeled with antibodies, and PMN-MDSC cells (CD45+/CD11b+/Ly6G+) were sorted. 10⁵ PMN-MDSC cells were added to the upper layer of Transwell and then placed in a 37°C incubator for 2hr, and then the number of PMN-MDSC cells in the lower layer was analyzed by flow assay to calculate the migration ratio of the cells.

6-3) PMN-MDSC in vivo cell migration assay: single-cell suspensions were prepared from isolated spleens of B16 tumor-bearing mice and labeled with antibodies, and PMN-MDSC cells (CD45+/CD11b+/Ly6G+) were sorted by flow cytometry. WT PMN-MDSC and CD300LD-KO PMN-MDSC were stained into high CTV labeling and low CTV labeling, respectively, by using CTV staining. WT PMN-MDSC and KO PMN-MDSC were mixed at a ratio of 1:1 and injected intravenously into tumor-bearing WT mice. Bone marrow, spleens, and tumors were isolated 24hr later, and the ratio of WT PMN-MDSC to CD300LD-KO PMN-MDSC was analyzed by flow cytometry.

6-4) T cell proliferation assay: spleens of OT-1 mice were isolated, single-cell suspensions in PBS (5×10⁷ cells/ml) were prepared, and CTV dye with a final concentration of 5µM was added to obtain a mixture. The mixture was incubated in a 37°C water bath for 20 minutes, added with 5 times the volume of ice precooled RPMI medium (RPMI-1640+10% FBS), mixed by inversion, stood at room temperature for 5 minutes, centrifuged for 5 minutes at 400g, and then resuspended in RPMI medium (1×10⁶ cells/ml). 80µl of CTV-labeled cells was added with 40µl of OVA peptide at a concentration of 100ng/ml, and then supplemented with 80 µl of RPMI medium. The solution was added to the wells of a 96-well U bottom plate and incubated at 37°C in a carbon dioxide incubator, and then antibody labeling and flow assay were carried out 48-72hr later.

6-5) T cell proliferation inhibition assay of PMN-MDSC: spleens of B16 tumor-bearing mice were isolated, single-cell suspensions were prepared and labeled with antibodies, and PMN-MDSC cells (CD45⁺/CD11b⁺/Lying6G⁺) were sorted by flow cytometry and resuspended in RPMI medium (1×10⁶ cells/ml). 80µl of CTV-labeled cells in 8-1) was added with 40µl of OVA peptide at a concentration of 100ng/ml, PMN-MDSC cells, and culture medium. The solution was added to the wells of a 96-well U bottom plate and incubated at 37°C in a carbon dioxide incubator, and then antibody labeling and flow assay were carried out 48-72hr later. During the above T cell proliferation experiments, different ratios of PMN-MDSC cells were added, and antibody labeling and flow assay were carried out 48-72hr later.

### Results:

RNAseq analysis of PMN-MDSC in CD300LD WT and KO mice in this embodiment showed that neutrophil migration-related signaling pathways were significantly downregulated after CD300LD knockout (FIGs. 6A and B). In vitro cell migration assay demonstrated that the migration ability of CD300LD-KO PMN-MDSC was significantly weaker than that of WT PMN-MDSC (FIG. 6C). The CD300LD WT and KO PMN-MDSCs were mixed at a ratio of 1:1 and injected into tumor-bearing mice for migration competition experiments, and the results showed that WT PMN-MDSC recruited in tumors were significantly higher than CD300LD-KO PMN-MDSC (FIGs. 6D-F). The above results indicated that CD300LD knockout inhibited the migratory ability of PMN-MDSC, resulting in a significant reduction of PMN-MDSC recruited by the tumor.

The effect of CD300LD on PMN-MDSC function was further analyzed. The results of the T cell proliferation assay showed that WT PMN-MDSC was able to inhibit T cell proliferation efficiently, while this ability was significantly weakened for CD300LD-KO PMN-MDSC (FIGs. 6G and H), indicating that the T cell inhibitory function of PMN-MDSC was positively regulated by CD300LD. The above results suggested that CD300LD knockout also inhibited the T cell suppressive function of PMN-MDSC.

### Embodiment 6 CD300LD regulates PMN-MDSC migration and function through S100A8/A9

To investigate the molecular mechanism of CD300LD in regulating PMN-MDSC, transcriptome sequencing was performed on WT and KO PMN-MDSC, and significantly different genes were analyzed in this embodiment to investigate which downstream genes are used by CD300LD to regulate cell migration and function of PMN-MDSC.

### Materials and Methods:

7-1) PMN-MDSC in vitro cell migration assay (Transwell): spleens of B16 tumor-bearing mice were isolated, single-cell suspensions were prepared and labeled with antibodies, and PMN-MDSC cells (CD45+/CD11b+/Lying6G+) were sorted by flow cytometry. 10⁵ PMN-MDSC cells were added to the upper layer of Transwell and then placed in a 37°C incubator, and then S100A8/A9 inhibitor (Tasquinimod) or DMSO control with a final concentration of 1µg/ml was added, the number of PMN-MDSC cells in the lower layer was analyzed by flow-assay after 2hr, and the percentage of cell migration was calculated.

7-2) Tumor model: B16 cells (1.5×10⁵ cells/mouse) were inoculated into mice subcutaneously, and 20 µg of Tasquinimod/mouse was injected intraperitoneally on day 2. Tumor size was measured every 2-3 days, and tumor growth curves were plotted. Tumors were isolated from mice on day 19, and the percentage of immune cells was determined by flow analysis.

### Results:

To further explore the molecular mechanism of CD300LD in regulating PMN-MDSC, RNA-seq difference analysis was performed on PMN-MDSC isolated from WT and KO mice, where S100A8/A9 was the most significantly down-regulated gene in the KO PMN-MDSCs (FIG. 7A) and was reported to be associated with the function of PMN-MDSC. S100A8/A9 levels in the serum of WT tumor-bearing mice were significantly greater than those in the serum of normal mice, whereas S100A8/A9 levels in KO tumor-bearing mice were almost the same as those in normal mice (FIG. 7B). Meanwhile, the expression levels of S100A8 and S100A9 in CD300LD-KO PMN-MDSC were also significantly lower than those in WT PMN-MDSC (FIG. 7C). In vitro migration assay demonstrated that S100A8/A9 proteins could enhance the weakened migration ability of KO PMN-MDSCs (FIG. 7D). T-cell inhibition assay showed that the S100A8/A9 inhibitor significantly reduced the inhibitory effect of WT PMN-MDSCs, but had little effect on KO PMN-MDSCs (FIG. 7E). The tumor model further showed that in vivo injection of the S100A8/A9 inhibitor Tasquinimod inhibited tumor growth in WT mice, but barely inhibited tumor growth in KO mice, and the proportion of intratumoral PMN-MDSCs was also consistent with the changes of tumors (FIGs. 7F and G). The above results demonstrated that CD300LD regulates the migration and function of PMN-MDSC through S100A8/A9, which are important effector molecules for downstream of CD300LD.

### Embodiment 7 CD300LD knockout and PD1 antibody have significant anti-tumor synergistic effects

CD300LD is mainly expressed on myeloid cells (i.e., PMN-MDSC) and PD1 is mainly expressed on T cells, the synergistic anti-tumor effects of these two molecules are investigated. The anti-tumor effect of CD300LD knockout combined with PD1 antibody was analyzed in this embodiment.

### Materials and Methods:

7-1) Expression analysis of PD1 and its ligand PD-L1: single-cell suspensions were prepared from isolated tumors of B16 mouse, and stained with PD1 and PD-L1 antibodies. PD1 and PD-L1 expression on the surface of tumor cells and immune cells were measured by flow analysis.

7-2) Tumor model: B16-F10 cells (1.5×10⁵ cells/mouse) were inoculated subcutaneously into WT and CD300LD KO mice, and PD1 antibody was injected intravenously at 200 µg/mouse on day 7 after inoculation for 3 times. Tumor size was measured every 2-3 days and tumor growth curves were plotted.

### Results:

Flow analysis results showed an elevated proportion of PD1⁺ to CD8⁺ T cells in the tumors of KO mice compared to WT mice, as well as an elevated proportion of PD-L1 expression in tumor cells and PMN-MDSC cells (FIG. 8A), suggesting CD300LD had a synergistic effect with PD1. The effect of the PD1 antibody under the B16-F10 tumor model in WT and CD300LD-KO mice was tested. The results showed that WT mice treated with PD1 antibody alone had the same effect as CD300LD KO, both of which significantly inhibited tumor development, and combined with CD300LD KO, PD1 antibody was able to further inhibit tumor development and prolong the survival time of the mice (FIGs. 8B and C). The above results demonstrated that CD300LD knockout and PD1 immune checkpoint had obvious anti-tumor synergistic effects.

### Embodiment 8 CD300LD knockout does not affect normal mouse development

In order to explore the systemic risks that may result from targeting CD300LD, the effect of CD300LD knockout on the development of the organism was analyzed and a preliminary assessment of the safety of CD300LD target was conducted.

### Materials and Methods:

8-1) Immune cell population analysis: mouse bone marrow, spleens, and peripheral blood were isolated, single-cell suspensions were prepared, and the proportion of different immune cell populations was analyzed using flow antibodies, where the immune cell populations include T cells (CD3⁺), B cells (B220⁺), myeloid cells (CD11b(Mac1)⁺), granulocytes (CD11b⁺Gr1⁺); LSK hematopoietic precursor cells (Lin⁻Sca1⁺cKit⁺), LT-HSC (Lin⁻Sca1⁺cKit⁺CD34⁻Flt3⁻), MMP (Lin⁻Sca1⁺cKit⁺CD34⁺Flt3⁺), GMP (Lin⁻cKit⁺Sca1⁻CD34^{High}FcyR^{High}), CMP (Lin⁻cKit⁺Sca1⁻CD34^{High}FcyR^{Mid}), MEP (Lin⁻cKit⁺Sca1⁻CD34^{Low}FcyR^{Low}), and CLP (Lin⁻cKit^{Mid}Sca1^{Mid}Flt3^{High}IL7R^{High}). Single-cell suspensions were prepared from tumors, stained using PD1 antibody and PD-L1 antibody, and analyzed for PD1 and PD-L1 expression on the surface of tumor cells as well as immune cells using flow cytometry.

8-2) CFU assay: mouse bone marrow was isolated, single-cell suspension was prepared, 10⁵ cells were cultured in cellulose semi-solid medium (MethoCult GF M3434, Stem Cell Inc.), and after 7 days of incubation, BFU-E, CFU-GM, and CFU-GEMM clones were counted under the microscope; or bone marrow cells were cultured in cellulose semi-solid medium (MethoCult GF M3634, Stem Cell Inc.), and after 7 days of incubation, Pre-B clones were counted under the microscope.

### Results:

CD300LD KO and WT mice grew and developed normally with no visible abnormal behaviors. Flow analysis showed that there were no significant differences in the proportions of T cells, B cells, and myeloid cells in the immune and hematopoietic organs of KO and WT mice (FIG. 9A), there were no significant differences in the proportions of long-term and short-term hematopoietic stem cells (LT-HSC, ST-HSC) and multipotent hematopoietic progenitors (MPP), and there were no significant differences in the proportions of other precursor cells, including the myeloid precursor cells, such as CMPs, GMPs, and MEPs, as well as lymphoid precursor cells (CLPs) (FIGs. 9B and C). Further analysis showed that there was no significant difference between the clone formation ability of hematopoietic stem cells in KO and WT mice (FIG. 9D). The above results indicated that the knockout of CD300LD does not affect the normal development of mice and the development of hematopoietic/immune system, suggesting that targeting CD300LD is safe.

### Embodiment 9 CD300LD knockout inhibits the development of established tumors

Whether induced CD300LD knockout can inhibit tumor development of established tumors is further analyzed in this embodiment.

### Materials and Methods:

Tumor model with induced CD300LD knockout: B16 cells (1.5 × 10⁵ cells/mouse) were inoculated subcutaneously into CD300LD^{fl/fl}Mx1CRE mice, and PolyIC was injected on day 7 after inoculation to induce CD300LD knockout once every 2 days for a total of 4 times. Tumor size was measured every 2-3 days and tumor growth curves were plotted.

### Results:

Using CD300LD^{fl/fl} mice bred with MxICRE mice, CD300LD^{fl/fl}Mx1CRE mice were generated, which allows for the knockout of CD300LD under PolyIC drug induction. CD300LDfl/flMx1CRE mice were inoculated with B16-F10 cells subcutaneously for tumor formation, and PolyIC treatment was performed when the tumors grew to about 100 mm³. The results showed that induced knockout of CD300LD significantly inhibited the development of established tumors (FIG. 10A), whereas there was no difference in tumor growth between the two groups of mice without drug administration (FIG. 10B). The above results suggested that for the established tumors, induced knockout of CD300LD was able to significantly inhibit tumor development, which also further indicated that CD300LD is an effective target for tumor immunotherapy.

### Embodiment 10 Construction and protein purification of mouse CD300LD-ECD

The Applicant's above results showed that CD300LD was a key receptor for PMN-MDSC to suppress tumor immunity, and the knockout of CD300LD significantly inhibited the development of multiple tumor models. The extracellular region of CD300LD was constructed for competitive inhibition of CD300LD and the role of CD300LD-ECD protein on tumor development was validated in this embodiment. This embodiment includes the construction and purification of mouse CD300LD-ECD protein.

### Materials and Methods:

1-1) construction of CD300LD-ECD-hFc fusion expression vector: PCR amplification of mouse CD300LD extracellular region fragments was performed first using the following PCR amplification primers
For: 5' tgcggccgcgaattcatgtggcagttctctgctctct 3' (SEQ ID NO. 14),
Rev: 5' cctgctgagcagcatctacgtcgacatcgagggc 3' (SEQ ID NO. 15)
PCR conditions: 95°C for 5min, (95°C for 30s, 58°C for 30s, 72°C for 60s, 30cycles), 72°C for 10min, 4°C for 10min.

The nucleotide sequence of the mouse CD300LD extracellular region fragment is shown as SEQ ID NO: 16, and the corresponding amino acid sequence is shown as SEQ ID NO:17. The fragment was inserted into the pCMV-hFc vector (with the nucleotide sequence shown as SEQ ID NO.18) using the one-step cloning reagent (NovoRec^{®} plus One step PCR Cloning Kit, Novoprotein, NR005-01A) according to the instructions.

1-2) Protein expression and purification: pCMV-mCD300LD-ECD-hFc plasmid (with the nucleotide sequence shown as SEQ ID NO.19) or pCMV-hFc plasmid was transfected into 293e cells using the PEI transfection method. 96 hours later, the cell supernatant was collected, filtered through a 0.45 µm filter, and purified by protein A affinity chromatography. The buffer was replaced by PBS using the PD-10 desalting column (GE), and finally the protein was concentrated to 1 mg/ml using the protein concentration column (AMICON).

### Results:

It was verified that the amino acid sequence of the mouse CD300LD extracellular region fragment obtained was consistent with the design. hFc was used as a control in this embodiment, and FIG. 11A showed the structures of CD300LD-ECD-hFc and hFc. FIG. 11B showed the SDS-PAGE results of purified CD300LD-ECD-hFc protein and hFc protein.

### Embodiment 11 Mouse CD300LD-ECD significantly inhibits the development of the established B16-F10 tumor model

B16-F10 tumor mouse model was utilized in this embodiment to assess the tumor inhibitory function of mouse CD300LD-ECD, specifically, CD300LD-ECD treatment was performed on established tumors to detect its effect on tumor development.

### Materials and Methods:

2-1) B16-F10 cell culture: B16-F10 cells were cultured in DMEM medium (10% FBS, 1% Penicillin-Streptomycin) at 37°C with 5% CO₂. The cells were passaged at a ratio of 1:3 every 2 days.

2-2) B16-F10 melanoma tumor model: B16-F10 cells (1.5×10⁵ cells/mouse) were inoculated subcutaneously into mice, the tumor size was measured every 2-3 days, and the tumor growth curves were plotted. Mice with a tumor volume of about 1000cm³ were deemed dead, and the survival curves of the mice were plotted.

2-3) CD300LD-ECD protein treatment of tumor-bearing mice: On day 7 after B16-F10 cell inoculation, the purified protein in Embodiment 10 was injected at a dose of 200 µg protein/mouse via the tail vein once every 2 days for a total of 4 times.

2-4) Tumor immuno-microenvironment analysis: single-cell suspensions were prepared from isolated mouse tumors and analyzed by flow assay after labeling with fluorescent antibodies, where neutrophils/PMN-MDSCs were stained with CD45⁺/CD11b⁺/Ly6G⁺ and CD8⁺T was stained with CD45⁺/CD3⁺/CD8⁺.

### Results:

On day 7 after tumor cell inoculation, the tumor size reached about 100 mm³ subcutaneously, and these mice were randomly divided into two groups, where one group was treated with CD300LD-ECD-hFc protein, and the other group was treated with hFc protein. The results showed that tumor development was significantly inhibited in the CD300LD-ECD-treated group, compared to the control hFc group (FIG. 12A). Applicant's above studies demonstrated that CD300LD knockout resulted in a significant improvement in the tumor microenvironment, including a significant reduction in PMN-MDSC and a significant increase in CD8⁺T infiltration. Therefore, the tumor microenvironment in the above mice was also analyzed in this embodiment. The results showed that there was a significant reduction in PMN-MDSC and a significant increase in CD8+T in the tumors after CD300LD-ECD treatment, compared to the control group (FIGs. 12B and C). The above results indicated that CD300LD-ECD can mimic CD300LD knockout and significantly improve the tumor immune microenvironment, thereby inhibiting tumor development.

### Embodiment 12 Upregulation of human CD300LD expression in multiple tumors

Applicant's above studies found that CD300LD expression was significantly upregulated in tumor-bearing mice. Whether human CD300LD gene expression changes in tumor patients was analyzed in this embodiment.

### Materials and Methods:

3-1) Immunohistochemistry of samples of melanoma patients: paraffin-embedded samples of melanoma patients were sectioned, labeled with anti-human CD300LD antibody (with a final concentration of 5 µg/ml) overnight at 4°C, rinsed in PBS for 3 minutes for a total of 3 times, added with biotin-labeled goat anti-human IgG secondary antibody (with a final concentration of 1 µg/ml) for incubation for 10 minutes at room temperature, rinsed in PBS for 3 minutes for a total of 3 times, added with HRP-Streptavidin (with a final concentration of 1µg/ml) for incubation for 10 minutes at room temperature, washed with PBS for 3 minutes for a total of 3 times, subjected to DAB staining, and observed under a microscope.

3-2) Establishment of CD300LD gene expression in immune cells in patient's blood: peripheral blood from patients with tumor was collected and the immune cells in the blood were isolated using Percoll according to the method known in the field. Total cellular RNA was extracted using Trizol reagent according to conventional methods in the field, and mRNAs were reversely transcribed to cDNAs using reverse transcriptase and oligo d(T)16 (Invitrogen, Cat#N8080128). The expression of CD300LD and GAPDH in the samples was quantitatively analyzed by qPCR, and the specific primers and conditions were as follows.
huLD-For: 5'TCCCAGGTTACTCCATTGCC 3' (SEQ ID NO.20)
huLD-Rev: 5'GCCTGAGCCATAAGCACACT 3' (SEQ ID NO.21)
huGAPDH-For: 5'TGTGGGCATCAATGGGATTTGG 3' (SEQ ID NO.22)
huGDPDH-Rev: 5'ACACACCATGTATTCCGGGGTCAAT 3' (SEQ ID NO.23)
qPCR conditions: 95°C for 5min, (95°C for 30s, 58°C for 30s, 72°C for 30s, 40cycles), 72°C for 10min, and 4°C for 10min.

3-3) Expression analysis of CD300LD in different tumors: the mRNA expression levels of CD300LD in different kinds of tumors and corresponding normal tissues were measured using the TCGA database.

### Results:

Immunohistochemistry of samples of melanoma patients showed that CD300LD was significantly expressed in tumor tissues, while rarely expressed in tumor-adjacent tissues (FIG. 13A). Fluorescence quantitative PCR was performed on peripheral blood immune cells from normal subjects and bowel cancer patients, and the results showed that the expression of CD300LD was significantly increased in the immune cells of bowel cancer patients (FIG. 13B). To further analyze the expression of CD300LD in other tumor types, the TCGA database was used, and it was found that the expression level of CD300LD in 23 kinds of tumors was higher than that of the corresponding normal tissues (FIG. 13C). The above results indicated that the expression of CD300LD was significantly upregulated in a variety of tumors.

### Embodiment 13 CD300LD signaling pathway negatively correlates with the survival rate of patients with melanoma tumor

The correlation between the CD300LD signaling pathway and the survival rate of patients with melanoma tumor was further analyzed in this embodiment.

### Materials and Methods:

Clinical information and gene expression levels of patients with melanoma tumor were obtained from the TCGA database, and the patients were divided into two groups (high group and low group) according to the median CD300LD expression level for survival correlation analysis (Graphpad software). In addition, the CD300LD downstream significant positively correlated genes (S100A8, NGP, LTF, S100A9, CAMP) were utilized, the expression value of each of these genes was standardized to form CD300LD signature values, and the patients were divided into two groups (high group and low group) according to the median value of the CD300LD signature values for the survival correlation analysis. The significance of the correlation was calculated using the Gehan-Breslow-Wilcoxon test.

### Results:

The results showed that among patients with melanoma tumor, the survival rate of the CD300LD high expression group was lower than that of the CD300LD low expression group (FIG. 14A). A comprehensive analysis was also performed using CD300LD signature consisting of significant genes positively regulated by CD300LD, and the results showed that the survival rate was significantly lower in the group with high expression of CD300LD signature (FIG. 14B). The above results indicated that the CD300LD signaling pathway is negatively correlated with the survival rate of patients with melanoma tumor.

### Embodiment 14 Construction of CD300LD humanized mice

To verify whether targeting human CD300LD can similarly inhibit tumor development, a tumor model was established in CD300LD humanized mice in this embodiment.

### Materials and Methods:

5-1) Construction and identification of CD300LD humanized mice: using CrisprCas9 technology, the first exon of CD300LD of C57BL6 mice was replaced with human CD300LD, and the construction of CD300LD humanized mice was entrusted to a third-party company GemPharmatec.

The following primers were used to identify whether the CD300LD humanized mice were successfully constructed.
WT-For, 5' TTAGAGGAAGTTCCCTGTTCACCC 3'; (SEQ ID NO.24)
WT-Rev, 5' CTTACCTGGGGAGGAAGAATAGGAG3 ; (SEQ ID NO.25)
Mutant-For, 5' TGCAGATTCCCACAATATGGGGAG 3'; (SEQ ID NO.26)
Mutant-Rev, 5' AACAGGAAGTGGGGTGCTCTTGAG 3'; (SEQ ID NO. 27)
PCR reaction conditions: 95°C for 5min, 30cycles (95°C for 30s, 58°C for 30s, 72°C for 30s), 72°C for 10min, and 4°C for 10min.

The WT band is 267 base pairs (bp), and the Mutant band is 1393 base pairs (bp).

5-2) B16-F10 melanoma tumor mouse model: B16-F10 cells (1.5×10⁵ cells/mouse) were inoculated subcutaneously into mice, the size of the tumor was measured every 2-3 days, and the tumor growth curves were plotted. Mice with a tumor volume of about 1000cm³ were deemed dead, and the survival curves of mice were plotted.

### Results:

FIGs. 15A and B showed that mouse CD300LD was successfully replaced by human CD300LD using CrisprCas9 technology, and human CD300LD mice were successfully constructed. The B16-F10 mouse models were constructed in wild-type mice, CD300LD knockout mice, and CD300LD humanized mice to analyze tumor development in different mice. The results showed that tumor development was significantly inhibited in CD300LD knockout mice compared to wild-type mice, and tumor development in CD300LD humanized mice was not different from that of wild-type mice, suggesting that human CD300LD is functionally equivalent to mouse CD300LD (FIG. 15C). This indicated that humanized mice can be used to evaluate the effects of drugs targeting human CD300LD.

### Embodiment 15 Human CD300LD-ECD significantly inhibits tumor development

The extracellular region protein of CD300LD (hCD300LD-ECD) was constructed and purified in this embodiment to competitively inhibit CD300LD in CD300LD humanized mice under a tumor model, so as to analyze its effect on tumor development.

### Materials and Methods:

6-1) Vector construction and expression purification of CD300LD-ECD-hFc fusion protein:
PCR amplification of the extracellular region fragment of human CD300LD was performed according to primers and conditions shown below:
For: 5' tgcggccgcgaattcgccaccatgtggctgtccccatc 3' (SEQ ID NO.28)
Rev: 5' gtccccgctcaagagcacccacgtcgacatcgagggc 3' (SEQ ID NO.29)
PCR conditions: 95°C for 5min, (95°C for 30s, 58°C for 30s, 72°C for 60s, 30cycles), 72°C for 10min, and 4°C for 10min.

The nucleotide sequence of the extracellular region fragment of human CD300LD is shown as SEQ ID NO.30, and the corresponding amino acid sequence is shown as SEQ ID NO.31. The fragment was inserted into the pCMV-hFc vector using the one-step cloning reagent (NovoRec^{®} plus One step PCR Cloning Kit, Novoprotein, NR005-01A) according to the instructions. pCMV-mCD300LD-ECD-hFc plasmid (with the nucleotide sequence shown as SEQ ID NO.32) or pCMV-hFc plasmid was transfected into 293e cells using the PEI transfection method. 96 hours later, the cell supernatant was collected, filtered through a 0.45 µm filter, and purified by protein A affinity chromatography. The buffer was replaced by PBS using the PD-10 desalting column (GE), and finally the protein was concentrated to 1 mg/ml using the protein concentration column (AMICON).

6-2) CD300LD-ECD protein treatment of tumor-bearing mice: B16-F10 cells (1.5×10⁵ cells/mouse) were inoculated subcutaneously into mice, on day 7 after inoculation, proteins were injected via tail vein at a dose of 200 µg protein/mouse once every 2 days for a total of 4 times.

### Results:

The hCD300LD-ECD-hFc fusion protein was constructed and purified (FIGs. 16A-B). The B16-F10 tumor model was established in CD300LD humanized mice. On day 7 after tumor inoculation, hCD300LD-ECD-hFc fusion protein was injected via tail vein at a dose of 200 µg protein/mouse, and hFc protein serves as a control. The results showed that hCD300LD-ECD-hFc significantly inhibited the development of established tumors (FIG. 16C).

### Embodiment 16 Human CD300LD-ECD and PD1 antibody have a significant antitumor synergistic effect

CD300LD is mainly expressed on myeloid PMN-MDSC cells and PD1 is mainly expressed on T cells. In this embodiment, whether targeting these two targets simultaneously will have a synergistic anti-tumor effect is studied.

### Materials and Methods:

7-1) Drug treatment: B16-F10 cells (1.5×10⁵ cells/mouse) were inoculated subcutaneously into CD300LD humanized mice. hCD300LD-ECD treatment: on day 7 after inoculation, the protein at a dose of 200 µg protein/mouse was injected into mice via the tail vein once every 2 days for a total of 4 times. PD1 antibody (BioXcell Inc. InVivoMab antimouse PD-1 (CD279), Cat#BE0273) Treatment: on day 8 after inoculation, the antibody at a dose of 200 µg protein/mouse was injected into mice via the tail vein once every 2 days for a total of 3 times. After tumor inoculation, the size of the tumor was measured every 2-3 days, and the tumor growth curves were plotted. Mice with a tumor volume of about 1000cm³ were deemed dead, and the survival curves of the mice were plotted.

### Results:

The results showed that when administered separately, hCD300LD-ECD and PD1 antibody both significantly inhibited tumor development. Furthermore, when used in combination, the tumor growth was further suppressed (FIG. 17A). Mouse survival curves also showed the same trend (FIG. 17B). The above results indicated that hCD300LD-ECD and PD1 antibody had obvious anti-tumor synergistic effects.

In summary, CD300LD plays a crucial role in the tumor immune microenvironment through PMN-MDSC. CD300LD-ECD competitively inhibits CD300LD, significantly restraining tumor development. It further demonstrates a pronounced anti-tumor synergistic effect when combined with PD1 antibody, offering a novel targeting drug for tumor immunotherapy.

The above embodiments are intended to illustrate the disclosed embodiments of the present disclosure and should not be construed as limitations of the present disclosure. In addition, the various modifications set forth herein, as well as variations in the methods of the present disclosure, will be apparent to those skilled in the art without departing from the scope and spirit of the present disclosure. While the present disclosure has been specifically described with reference to a variety of specific preferred embodiments of the present disclosure, it should be understood that the present disclosure is not be limited to these specific embodiments. Indeed, a variety of modifications of the present disclosure as described above that would be obvious to one of skill in the art should be included within the scope of the present disclosure.

## Claims

1. A use of CD300LD gene as a target in the preparation of tumor preventive, diagnostic, or therapeutic drugs.

2. A use of CD300LD gene and PD1 together as targets in the preparation of tumor preventive, diagnostic, or therapeutic drugs.

3. A use of a CD300LD inhibitor in the preparation of a product having at least one of the following effects:
1) tumor prevention, diagnosis, or treatment;
2) regulation of myeloid cell activity and/or migration;
3) regulation of T cell activity; and
4) regulation of CD300LD downstream signaling pathway.

4. The use according to claim 3, wherein the CD300LD inhibitor targets CD300LD Exon2, and/or, the CD300LD inhibitor is selected from a nucleic acid molecule, a protein molecule, and a small molecule compound.

5. The use according to claim 3, wherein the tumor is a CD300LD-overexpressing tumor; preferably, the tumor is selected from an intestinal cancer, a lung cancer, a liver cancer, a breast cancer, an esophageal cancer, a head and neck cancer, a skin cancer, a kidney cancer, a leukemia, a cervical cancer, a colon cancer, a hepatocellular carcinoma, an ovarian serous cystadenocarcinoma, an endometrial carcinoma, a thyroid cancer, a cutaneous melanoma, a lung adenocarcinoma, a head and neck squamous cell carcinoma, a glioblastoma multiforme, a prostate cancer, a thymic carcinoma, a brain low-grade glioma, a rectal adenocarcinoma, a pheochromocytoma and paraganglioma, an esophageal carcinoma, a renal clear cell carcinoma, a bladder urothelial carcinoma, a renal papillary cell carcinoma, a pancreatic cancer, a stomach cancer, a chromophobe renal cell carcinoma, a breast invasive carcinoma, a lung squamous cancer, a sarcoma, and an acute myeloid leukemia.

6. The use according to claim 3, wherein the myeloid cell is selected from macrophages, M-MDSC, and PMN-MDSC.

7. The use according to claim 3, wherein the regulation of myeloid cell activity is selected from reducing the number of myeloid cells, and causing a decrease in the expression of genes related to suppression of immunity on myeloid cells.

8. The use according to claim 3, wherein the regulation of T cell activity is selected from any one or more of the following: increasing the number of CD4+ and CD8+ T cells; decreasing the number of Treg cells; increasing the expression of genes related to T cell killing function; and increasing the expression of genes related to T cell recruitment.

9. The use according to claim 3, wherein the regulation of CD300LD downstream signaling pathway comprises regulating S100A8/A9 gene in the CD300LD downstream signaling pathway.

10. A method for in vitro screening of drugs, comprising the following steps:
mixing CD300LD-overexpressed cells with a drug to be screened, and monitoring the changes of the cells.

11. An isolated polypeptide, comprising a functional fragment of a CD300LD protein, wherein the functional fragment of the CD300LD protein is an extracellular region of the CD300LD protein.

12. The isolated polypeptide according to claim 11, wherein the extracellular region of the CD300LD protein is derived from a mammalian CD300LD protein or a mutant thereof; wherein the mammal is selected from a rodent, an even-toed ungulate, an odd-toed ungulate, a lagomorph, and a primate.

13. The isolated polypeptide according to claim 12, wherein the rodent is a mouse; and/or, the primate is selected from monkeys, apes, or humans.

14. The isolated polypeptide according to claim 11, wherein an amino acid sequence of the extracellular region of the CD300LD protein is shown as SEQ ID NO. 17 or SEQ ID NO. 31.

15. The isolated polypeptide according to claim 11, wherein the isolated polypeptide further comprises a protein purification tag.

16. The isolated polypeptide according to claim 11, wherein an amino acid sequence of the isolated polypeptide is shown as SEQ ID NO.33 or SEQ ID NO.34.

17. A nucleic acid construct, wherein the nucleic acid construct encodes the isolated polypeptide of any one of claims 11-16.

18. The nucleic acid construct according to claim 17, wherein the nucleic acid construct comprises a polynucleotide shown as SEQ ID NO.16 or SEQ ID NO.30.

19. The nucleic acid construct according to claim 17, wherein a nucleotide sequence of the nucleic acid construct is shown as SEQ ID NO.19 or SEQ ID NO.32.

20. A cell line, comprising the nucleic acid construct of any one of claims 17-19, or a genome of the cell line has an exogenous polynucleotide shown as SEQ ID NO. 16 or SEQ ID NO. 30.

21. A method for the preparation of the isolated polypeptide of any one of claims 11-16, comprising the following steps:
a) culturing the cell line of claim 10 under expression conditions, so as to express the isolated polypeptide of any one of claims 11-16;
b) isolating and purifying the polypeptide described in step a).

22. A use of the isolated polypeptide according to any one of claims 11-16 in the preparation of tumor preventive, diagnostic, or therapeutic drugs.

23. The use according to claim 22, wherein the tumor is a CD300LD-overexpressing tumor; preferably, the tumor is selected from an intestinal cancer, a lung cancer, a liver cancer, a breast cancer, an esophageal cancer, a head and neck cancer, a skin cancer, a kidney cancer, a leukemia, a cervical cancer, a colon cancer, a hepatocellular carcinoma, an ovarian serous cystadenocarcinoma, an endometrial carcinoma, a thyroid cancer, a melanoma, a lung adenocarcinoma, a head and neck squamous cell carcinoma, a glioblastoma multiforme, a prostate cancer, a thymic carcinoma, a brain low-grade glioma, a rectal adenocarcinoma, a pheochromocytoma and paraganglioma, an esophageal carcinoma, a renal clear cell carcinoma, a bladder urothelial carcinoma, a renal papillary cell carcinoma, a pancreatic cancer, a stomach cancer, a chromophobe renal cell carcinoma, a breast invasive carcinoma, a lung squamous cancer, a sarcoma, and an acute myeloid leukemia.

24. The use of the isolated polypeptide of any one of claims 11-16 and anti-PD1 antibody in the preparation of tumor preventive, diagnostic, or therapeutic drugs.

25. A composition, comprising a CD300LD inhibitor, wherein the composition has any one or more of the following effects:
1) tumor prevention, diagnosis, or treatment;
2) regulation of myeloid cell activity and/or migration;
3) regulation of T cell activity; and
4) regulation of CD300LD downstream signaling pathway.

26. The composition according to claim 25, wherein the composition is a pharmaceutical composition; and/or, the CD300LD inhibitor is the isolated polypeptide of any one of claims 11-16.

27. A method for treating a CD300LD-overexpressing disease, comprising administering to an individual a therapeutically effective amount of the isolated polypeptide of any one of claims 11-16, or the composition of claim 25.

28. The method according to claim 27, wherein the CD300LD overexpressing disease is a tumor; preferably, the tumor is selected from an intestinal cancer, a lung cancer, a liver cancer, a breast cancer, an esophageal cancer, a head and neck cancer, a skin cancer, a kidney cancer, a leukemia, a cervical cancer, a colon cancer, a hepatocellular carcinoma, an ovarian serous cystadenocarcinoma, an endometrial carcinoma, a thyroid cancer, a melanoma, a lung adenocarcinoma, a head and neck squamous cell carcinoma, a glioblastoma multiforme, a prostate cancer, a thymic carcinoma, a brain low-grade glioma, a rectal adenocarcinoma, a pheochromocytoma and paraganglioma, an esophageal carcinoma, a renal clear cell carcinoma, a bladder urothelial carcinoma, a renal papillary cell carcinoma, a pancreatic cancer, a stomach cancer, a chromophobe renal cell carcinoma, a breast invasive carcinoma, a lung squamous cancer, a sarcoma, and an acute myeloid leukemia.
